# EUROPEAN PATENT APPLICATION

(11) **EP 3 862 430 A1**
(43) Date of publication of application: **11.08.2021**
(21) Application number: 19869796.3
(22) Date of filing: 04.10.2019
(51) Int. Cl.: C12N 15/09, A01H 1/00, C12N 15/29, C12N 15/54, C12N 15/74

(54) **DNA CONSTRUCT TO BE USED IN GENOME EDITING OF PLANT**

(30) Priority: 04.10.2018 JP 2018188798
(71) Applicant: Kaneka Corporation, Osaka-shi, Osaka 530-8288 (JP)
(72) Inventor: NAGIRA, Yozo, Takasago-shi, Hyogo 676-8688 (JP); MIKI, Ryuji, Takasago-shi, Hyogo 676-8688 (JP); HAMADA, Haruyasu, Takasago-shi, Hyogo 676-8688 (JP); TAOKA, Naoaki, Takasago-shi, Hyogo 676-8688 (JP); EBINUMA, Hiroyasu, Ueda-shi, Nagano 386-8567 (JP)
(74) Representative: Hoffmann Eitle
(86) International application number: PCT/JP2019/039265
(87) International publication number: WO 2020/071528

(57) **Abstract**

A DNA construct for expressing a genome editing system in a plant cell, comprising: a cleaving target region flanked by two recombinase recognition sites arranged in the same direction, wherein a gene encoding a site-specific recombinase that specifically recognizes these recombinase recognition sites is arranged outside of the cleaving target region, the cleaving target region contains: a marker gene divided into a 5' region and 3' region; and a gene encoding the genome editing system arranged between the 3' region and the 5' region.

SEQUENCE LISTING

<110>  KANEKA CORPORATION

<120>  DNA CONSTRUCT TO BE USED IN GENOME EDITING OF PLANT

<130>  IBPF19-532WO

<150>  JP2018-188798
<151>  2018-10-04

<160>  36

<170>  PatentIn version 3.5

<210>  1
<211>  204
<212>  DNA
<213>  Artificial Sequence

<220>
<223>   lox-intron(KOR1)01

<400>  1
gtaagtcttc ttttccttta ctctttacag aaatggtaat ctcagatata gtaaataact      60

tcgtataatg tatgctatac gaagttattg gataagatcc aaaaatgaca cttttaacca     120

agattgtacg aagatctttt taaactccat tttttatttt gacatctaaa ttggatttaa     180

ctcggccttg ctgtattttg gcag                                            204

<210>  2
<211>  204
<212>  DNA
<213>  Artificial Sequence

<220>
<223>   lox-intron(KOR1)02

<400>  2
gtaagtcttc ttttccttta ctctttacag aaatggtaat ctcagatata gtaatggata      60

agatccaaaa atgacacttt taaccaaata acttcgtata atgtatgcta tacgaagtta     120

tgattgtacg aagatctttt taaactccat tttttatttt gacatctaaa ttggatttaa     180

ctcggccttg ctgtattttg gcag                                            204

<210>  3
<211>  30
<212>  DNA
<213>  Artificial Sequence

<220>
<223>  Ri-NPT-F

<400>  3
agctcggcac aagatgatcc tacaaggtag                                       30

<210>  4
<211>  30
<212>  DNA
<213>  Artificial Sequence

<220>
<223>  Ri-NPT-R

<400>  4
aaagagcgtt tagatgttgc catgttttac                                        30


<210>  5
<211>  30
<212>  DNA
<213>  Artificial Sequence

<220>
<223>  lox-GUS-F

<400>  5
ggatccgaat tccccgtccc cagattagcc                                        30


<210>  6
<211>  30
<212>  DNA
<213>  Artificial Sequence

<220>
<223>  Out-SmaI(GF-I)-R

<400>  6
acttcctgca ggcccctgcc aaaatacagc                                        30


<210>  7
<211>  21
<212>  DNA
<213>  Artificial Sequence

<220>
<223>  lox-iaaH-F

<400>  7
gtccccagat tagccttttc a                                                 21


<210>  8
<211>  27
<212>  DNA
<213>  Artificial Sequence

<220>
<223>  Out-SmaI(HSP-T)-R

<400>  8
acttcctgca ggcttatctt taatcat                                           27


<210>  9
<211>  30
<212>  DNA

<213>  Artificial Sequence

<220>
<223>  Out-KpnI(I-P)-F(end)

<400>  9
gatagtttaa actgggtaag tcttcttttc                                        30


<210>  10
<211>  30
<212>  DNA
<213>  Artificial Sequence

<220>
<223>  Out-KpnI(HSP-T)-R

<400>  10
aagcttgcgg ccgcgcttat ctttaatcat                                        30


<210>  11
<211>  30
<212>  DNA
<213>  Artificial Sequence

<220>
<223>  lox-ipt-F

<400>  11
gcttggatcc gaattgtccc cagattagcc                                        30


<210>  12
<211>  30
<212>  DNA
<213>  Artificial Sequence

<220>
<223>  lox-ipt-R

<400>  12
ggctaatctg gggaccttat ctttaatcat                                        30


<210>  13
<211>  30
<212>  DNA
<213>  Artificial Sequence

<220>
<223>  lox-iaaH-R

<400>  13
tggggacggg gaattcttat ctttaatcat                                        30


<210>  14
<211>  21
<212>  DNA
<213>  Artificial Sequence

<220>

<223>  lox-SmaI(HSP-T)-R

<400>  14
cttatcttta atcatattcc a                                                21


<210>  15
<211>  30
<212>  DNA
<213>  Artificial Sequence

<220>
<223>  lox-SmaI(HSP\201]T/ipt)-F

<400>  15
atgattaaag ataagcaacg aaggtaatgg                                       30


<210>  16
<211>  30
<212>  DNA
<213>  Artificial Sequence

<220>
<223>  lox-EcoRI(a-ipt-T)-R

<400>  16
ttatcctgca ggcccacgaa tatccgtgac                                       30


<210>  17
<211>  30
<212>  DNA
<213>  Artificial Sequence

<220>
<223>  Out-HindIII(35S-P)-F

<400>  17
gcgcggccgc aagctgtccc cagattagcc                                       30


<210>  18
<211>  30
<212>  DNA
<213>  Artificial Sequence

<220>
<223>   lox-HindIII(HSP-T)-R

<400>  18
attcggatcc aagctcttat ctttaatcat                                       30


<210>  19
<211>  30
<212>  DNA
<213>  Artificial Sequence

<220>
<223>  lox-NotI(cas9-P)-F

<400>  19

gatggtaccg cggccctagc aacgattgta                                        30

<210>  20
<211>  30
<212>  DNA
<213>  Artificial Sequence

<220>
<223>  lox-NotI(SgRNA-T)-R

<400>  20
ggacagcttg cggcccaact ttgtacaaga                                        30

<210>  21
<211>  23
<212>  DNA
<213>  Artificial Sequence

<220>
<223>  PDS3 (WT)

<400>  21
gaggcaagag atgtcctagg tgg                                               23

<210>  22
<211>  16
<212>  DNA
<213>  Artificial Sequence

<220>
<223>  PDS3 (d39-1)

<400>  22
gaggcaagag aggtgg                                                       16

<210>  23
<211>  23
<212>  DNA
<213>  Artificial Sequence

<220>
<223>  PDS3 (d39-2)

<400>  23
gaggcaagaa aggtggaagg tga                                               23

<210>  24
<211>  23
<212>  DNA
<213>  Artificial Sequence

<220>
<223>  PDS3 (d39-3)

<400>  24
gaggcaagag atgtcctgga tga                                               23

<210>  25
<211>  13
<212>  DNA
<213>  Artificial Sequence

<220>
<223>  PDS3 (d45-1)

<400>  25
gaggcaagag atg                                                          13


<210>  26
<211>  19
<212>  DNA
<213>  Artificial Sequence

<220>
<223>  PDS3 (d45-2)

<400>  26
gaggcaagag atgtccttt                                                    19


<210>  27
<211>  27
<212>  DNA
<213>  Artificial Sequence

<220>
<223>  NtPDS(1866-1892SG)F

<400>  27
acatataggg gttgcactct tctaagg                                           27


<210>  28
<211>  25
<212>  DNA
<213>  Artificial Sequence

<220>
<223>  NtPDS(2993-3017c)R

<400>  28
cccagtctca taccaatctc catca                                             25


<210>  29
<211>  34
<212>  DNA
<213>  Artificial Sequence

<220>
<223>  PDS3-F2(Affrc)

<400>  29
gtattgtcat caactggttg attatctgag tacc                                   34


<210>  30
<211>  28
<212>  DNA

<213>  Artificial Sequence

<220>
<223>  PDS3-R2(Affrc)

<400>  30
ctttccatat cttttaccta atatgctg                                          28


<210>  31
<211>  17
<212>  DNA
<213>  Artificial Sequence

<220>
<223>  PUC M4

<400>  31
gttttcccag tcacgac                                                      17


<210>  32
<211>  17
<212>  DNA
<213>  Artificial Sequence

<220>
<223>  PUC RV

<400>  32
caggaaacag ctatgac                                                      17


<210>  33
<211>  30
<212>  DNA
<213>  Artificial Sequence

<220>
<223>  pRI-GFP F

<400>  33
cactgttgat acatatggtg agcaagggcg                                        30


<210>  34
<211>  30
<212>  DNA
<213>  Artificial Sequence

<220>
<223>  pRI-GFP R

<400>  34
cttcatcttc ataagttact tgtacagctc                                        30


<210>  35
<211>  20
<212>  DNA
<213>  Artificial Sequence

<220>

<223>  FFCas9-113F

<400>  35
ccgataggca ctctatcaag                                                   20


<210>  36
<211>  20
<212>  DNA
<213>  Artificial Sequence

<220>
<223>  FFCas9-640R

<400>  36
cagagaggat agccttagca                                                   20

## Description

### [Technical Field]

The present invention relates to a DNA construct for expressing a genome editing system in a plant cell, and a method for producing a genome-edited plant cell using the DNA construct.

### [Background Art]

As tools for genetically modifying agricultural crops, genome editing technologies are attracting increasing attention worldwide. Genome editing technologies are technologies for rewriting (editing) a gene of interest (target gene) with pinpoint accuracy by using a genome editing system (CRISPR/Cas system, TALEN system, ZFN system, PPR system, or the like) formed of a domain binding specifically to a specific nucleic acid sequence on a genome and an enzyme domain for cleavage and the like of DNA. Since modification of target genes can be conducted simply and with high precision, genome editing technologies are increasingly utilized in various fields regarding plants such as breeding of agricultural crops, production of useful materials, cultivar improvement, analysis of functions of target genes, and the like (NPL 1).

However, even genome editing technologies have a problem that exogenous genes encoding genome editing systems, selection markers, and the like remain in genomes of the obtained genome-edited plants. For this reason, when it is desired to introduce only a necessary mutation into a plant genome, it is necessary to inhibit insertion of these genes into the genome or to remove these genes from the genome after insertion.

### [Citation List]

### [Non Patent Literature]

[NPL 1] OsakabeY. etal., Plant Cell Physiol., 2015, March, 56(3), 389-400
[NPL 2] Nanto,K. et al., Plant Biotechnol. J., 2005, 3, 203-214

### [Summary of Invention]

### [Technical Problem]

The present invention has been made in view of the above-described problem of the conventional technologies, and an object of the present invention is to provide a method for genome-editing of a plant that can modify a target gene without exogenous genes encoding a genome editing system and the like being incorporated into the plant genome.

### [Solution to Problem]

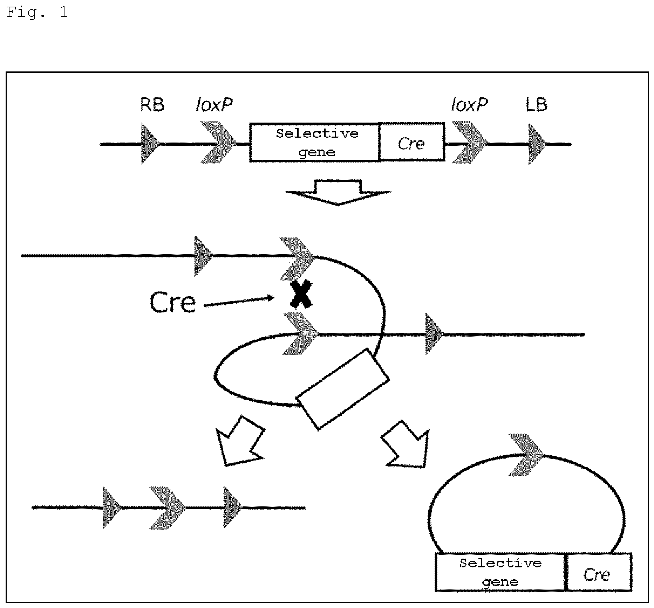

The present inventors focused on previously-developed MAT (Multi-Auto-Transformation) vector in order to achieve the above-described object. The MAT vector has an advantage that the MAT vector does not allow a marker gene used for selection (selective gene) to remain in the genome of the genome-edited plant by utilizing the Cre/loxP system, which is a site-specific recombination system. Specifically, as shown in Fig. 1, it has been revealed that in MAT vector, the selective gene incorporated in the genome is flanked by two recombinase recognition sites (loxP sequences), and the selective gene was cyclically cleaved by the action of the site-specific recombinase (Cre recombinase) (NPL 2).

In view of this, the present inventors made the concept of a system in which by incorporating an artificial restriction enzyme gene used for genome editing (gene encoding a genome editing system) or the like between the recombinase recognition sites instead of a selective gene, and transiently expressing the site-specific recombinase, it is possible to cleave the region containing the gene or the like and thus generate a cyclic DNA before incorporation into the genome. Moreover, the present inventors also made the concept that with divided marker genes being arranged at both end portions of the cleaved-off region, in the case where the cleaved-off region was circularized, the marker gene is reconfigured to become expressible, from which the behavior of the cyclic DNA is monitored.

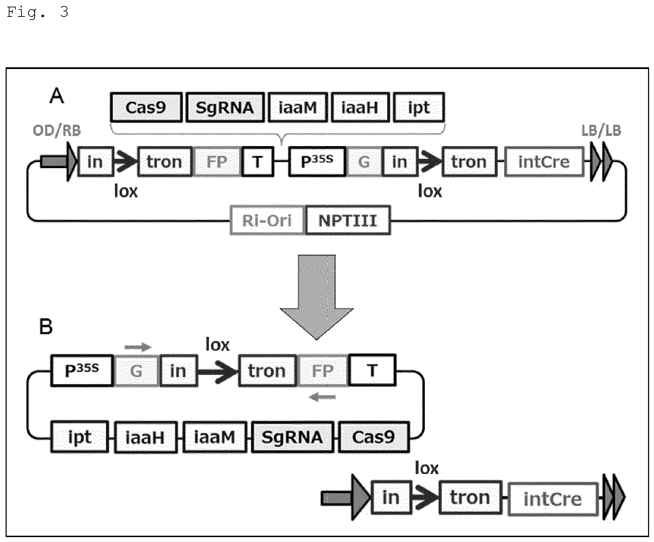

In fact, as a result of preparing a DNA construct shown in Fig. 3 and introducing the DNA construct into a plant, it was revealed that a cyclic DNA containing a gene encoding a genome editing system (SgRNA and Cas9) can be generated to allow the genome editing system to transiently express without the gene being incorporated into the plant genome, thus modifying the sequence of the target gene. Moreover, the present inventors also found that the cyclic DNA can be stably held for a certain period of time (about 35 days) in the plant cell, and consequently completed the present invention. Therefore, the present invention provides the following:
[1] A DNA construct for expressing a genome editing system in a plant cell, comprising:
   a cleaving target region flanked by a first recombinase recognition site located on a 5' side and a second recombinase recognition site located on a 3' side, wherein
   the first recombinase recognition site and the second recombinase recognition site are in the same direction,
   a gene encoding a site-specific recombinase that specifically recognizes the first and second recombinase recognition sites is arranged outside of at least one side of the cleaving target region,
   the cleaving target region contains: a gene encoding the genome editing system; and a marker gene divided into a 5' region and a 3' region,
   the 3' region of the marker gene is adjacent to a 3' terminal of the first recombinase recognition site, the 5' region of the marker gene is adjacent to a 5' terminal of the second recombinase recognition site, and the gene encoding the genome editing system is arranged between the 3' region and the 5' region of the marker gene, and
   when the DNA construct is introduced into a plant cell, the site-specific recombinase expresses to cleave out and circularize the cleaving target region, causing the 5' region and the 3' region to bind vie the recombinase recognition site, which allows the marker gene to express.
[2] The DNA construct according to [1], further comprising:
   a right boundary sequence (RB) and a left boundary sequence (LB) that are derived from an agrobacterium T-DNA sequence, wherein
   the cleaving target region and the gene encoding the site-specific recombinase are arranged in a transfer region flanked by the RB and the LB.
[3] The DNA construct according to [1] or [2], wherein
   the marker gene is a fluorescent protein gene, a luminescent enzyme gene, a chromogenic enzyme gene, or a drug resistance gene.
[4] The DNA construct according to any one of [1] to [3], wherein
   the marker gene is divided into the 5' region and the 3' region in intron.
[5] The DNA construct according to any one of [1] to [4], further comprising:
   an exogenous gene between the 3' region and the 5' region of the marker gene in the cleaving target region.
[6] The DNA construct according to [5], wherein
   the exogenous gene is a phytohormone biosynthetic gene.
[7] A method for producing a genome-edited plant cell, the method comprising the steps of:
   (1) introducing the DNA construct according to any one of [1] to [6] into a plant cell; and
   (2) selecting a cell in which the DNA construct has been introduced and circularization of the cleaving target region has occurred from cells obtained in the step (1) using expression of the marker gene as an indicator.
[8] A method for producing a genome-edited plant, the method comprising the steps of:
   (1) introducing the DNA construct according to [6] into a plant cell;
   (2) selecting a cell in which the DNA construct has been introduced and circularization of the cleaving target region has occurred from cells obtained in the step (1) using expression of the marker gene as an indicator; and
   (3) culturing the cell obtained in the step (2) to generate plant tissues, and selecting a plant tissue in which morphological change has occurred, using the morphological change in the plant tissues attributable to expression of the phytohormone biosynthetic gene on the DNA construct as an indicator.

### [Advantageous Effects of Invention]

The present invention makes it possible to modify the sequence of a target gene without exogenous genes encoding genome editing system and the like being incorporated into the genome in a plant cell. In addition, the present invention allows the genome editing system to stably express from the cyclic DNA. Therefore, it is possible to produce genome-edited plants having a high safety with high efficiency.

### [Brief Description of Drawings]

[Fig. 1] Fig. 1 is a diagram showing the summary of an MAT (Multi-Auto-Transformation) vector system, which serves as the base for the DNA construct of the present invention. In MAT vector, loxP sequences are located in the forward direction, between which selective gene and Cre recombinase, which is a site-specific enzyme, are located. Hence, the selective gene was cyclically cleaved by the Cre/loxP system, and a marker-free plant can be obtained.

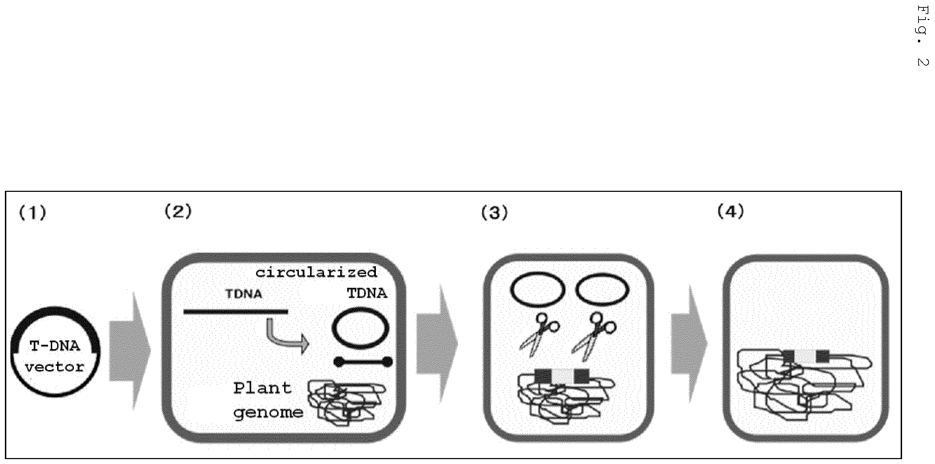

[Fig. 2] Fig. 2 is a diagram showing an example of genome editing using the DNA construct of the present invention. In the DNA construct of the present invention, arranging the site-specific enzyme, Cre gene in T-DNA and arranging its 2 recognition sites loxP in the forward direction cause the Cre/loxP system to cleave out the cyclic DNA when the DNA construct is introduced into plant cells. Moreover, incorporating Cas9, which is an artificial restriction enzyme, and SgRNA, which is the target sequence, (represented by scissors in the figure) in a region to be cyclically cleaved out in advance enables genome editing by cyclic DNA ("circularized DNA" in the figure) , which is not incorporated into the genome. Eventually, during cell division, cyclic DNAs disappear, making it possible to obtain mutated individuals in which no exogenous gene is incorporated into the genome.
[Fig. 3] Fig. 3 is a schematic diagram showing an aspect of the DNA construct of the present invention (A in the figure) and the cyclic DNA (B in the figure) to be cleaved out of the DNA construct. From the T-DNA region overdrive sequence (region flanked by "OD/RB" (overdrive sequence and right boundary sequence in the figure) and LB/LB (2 left boundary sequences) ) introduced by the agrobacterium method, the cleaving out of the cyclic DNA is caused by the Cre/loxP system. In the cyclic DNA, genes encoding the genome editing system ("Cas9" and "SgRNA" in the figure) and phytohormone biosynthetic genes ("iaaM", "iaaH", and "ipt" in the figure) are arranged. Non-recombinant mutagenesis can be conducted by the gene in the cyclic DNA transiently expressing in plant cells. In addition, the gfp gene is incorporated while divided in the DNA construct of the present invention ("P35S", "G", and "in", as well as "tron", "FP", and "T" in the figure) , which has such a mechanism that when circularization occurs, intron ("in" and "tron" in the figure) between the divided parts of the gfp gene rebinds and splicing occurs in plant cell, causing intron to be pulled out to allow encoded GFP protein to express. Note that "SgRNA" in the figure indicates a gene encoding the guide RNA for the target sequence in genome editing. "P35S" indicates the 35S cauliflower mosaic virus promoter. "T" indicates the heat shock protein terminator. "intCre" indicates the Cre recombinase gene in which intron was added at 5'. "lox" indicates the loxP sequence that Cre recombinase recognizes. "Ri-Ori" and "NPTIII" indicate kanamycin resistance genes. The arrows written together with "G" and "FP" indicate the primer sets for gfp gene detection. The descriptions in this figure also apply to the following figures.

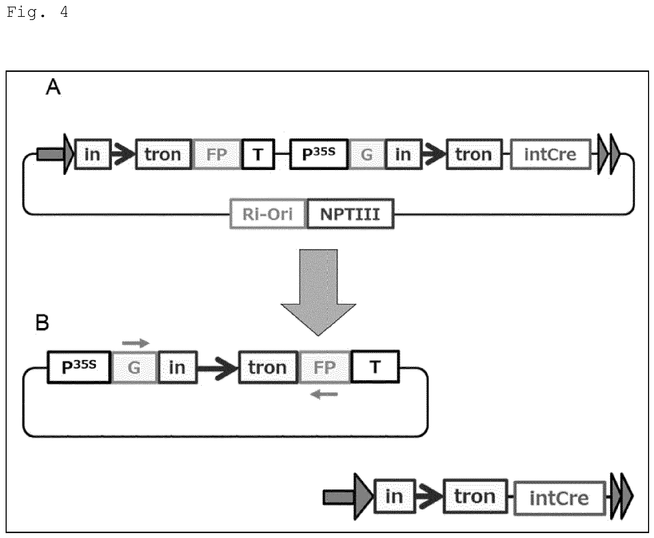

[Fig. 4] Fig. 4 is a schematic diagram of a vector prepared for verifying the activation of the DNA construct of the present invention.

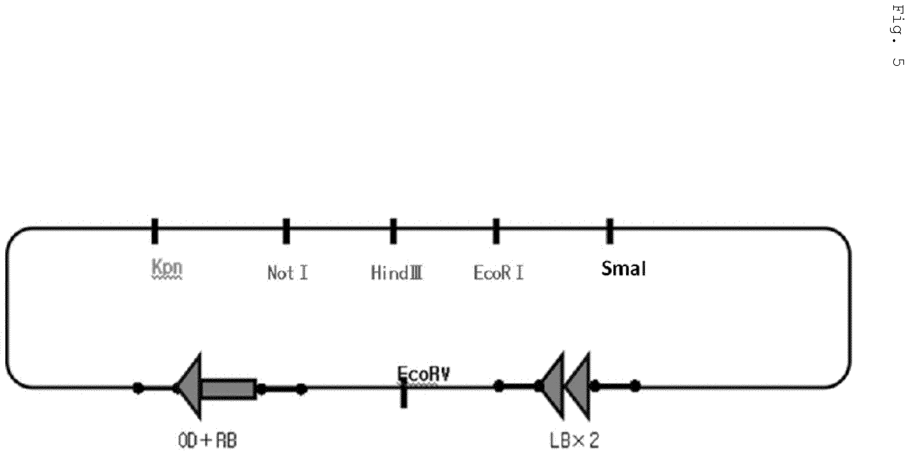

[Fig. 5] Fig. 5 is a schematic diagram showing a vector backbone for preparing the DNA construct of the present invention. The DNA construct of the present invention was prepared by inserting gene cassettes amplified with PCR into restriction enzyme sites in MCS located in the backbone by the in fusion reaction.

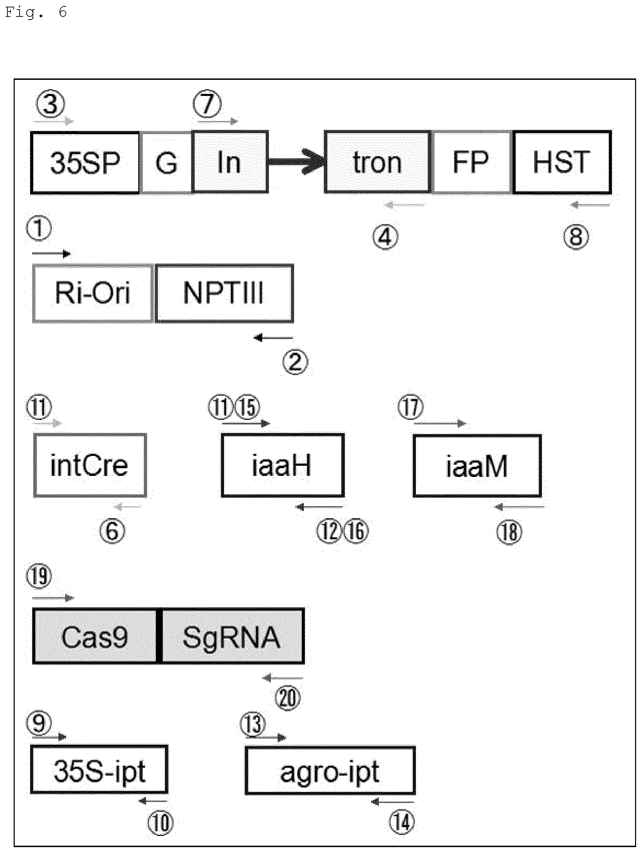

[Fig. 6] Fig. 6 is a schematic diagram showing gene cassettes inserted into the backbone shown in Fig. 5 and primers (the respective arrows in the figure) used for amplifying the gene cassettes.

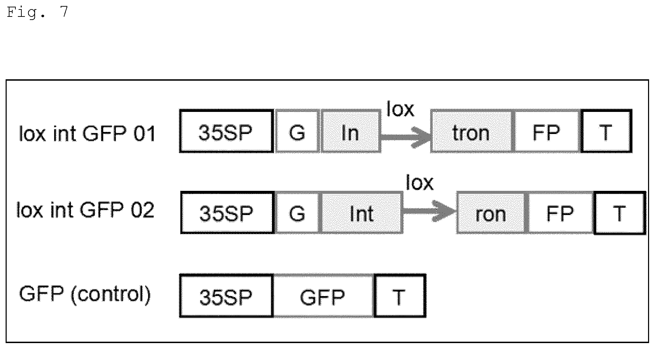

[Fig. 7] Fig. 7 is a schematic diagram showing the DNA constructs of the present invention that were used for agroinfiltration into Nicotiana benthamiana. For the agroinfiltration, two types of lox int GFP vectors different in position of loxP sequence ("lox" in the figure) in intron and a GFP vector having no intron as control were used. In the T-DNA region, 35S cauliflower mosaic virus promoter ("35SP" in the figure), heat shock protein terminator ("T" in the figure), and gfp ("G" and "FP", or "GFP" in the figure) are located.

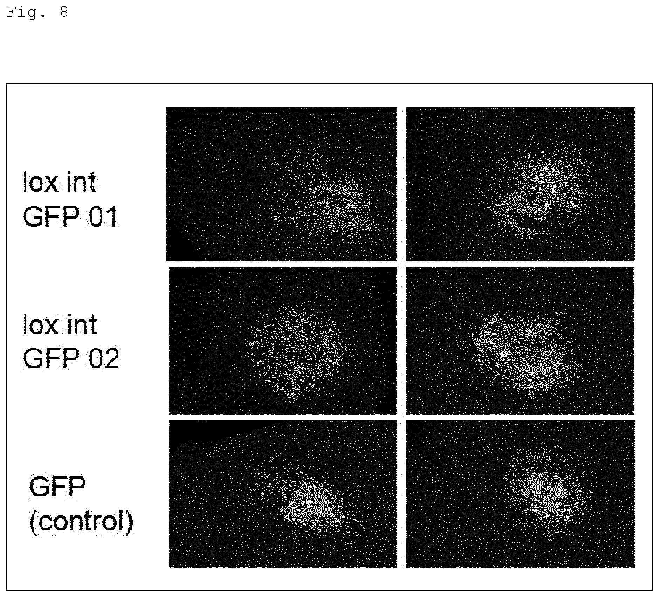

[Fig. 8] Fig. 8 is pictures showing the results of observing leaf pieces of Nicotiana benthamiana into which the lox intron GFP vector was introduced with fluorescence microscope. More specifically, Fig. 8 shows the results of observing GFP fluorescence at two positions in each leaf piece on day 6 from agroinfiltration into Nicotiana benthamiana. The upper stage shows lox int GFP 01 vector-derived GFP fluorescence, the middle stage shows lox int GFP 02 vector-derived GFP fluorescence, and the lower stage shows GFP vector-derived GFP fluorescence used as positive control.

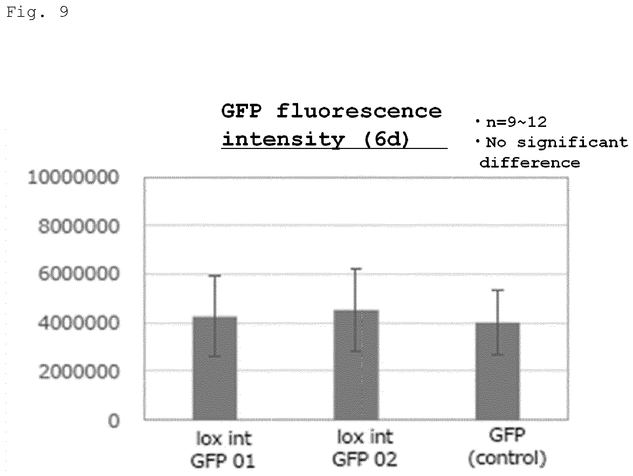

[Fig. 9] Fig. 9 is a graph showing the results of detecting fluorescence intensity in leaf pieces of Nicotiana benthamiana into which the lox intron GFP vector was introduced. More specifically, Fig. 9 shows the results of quantifying the GFP fluorescence intensity, for each leaf piece on day 6 shown in Fig. 8, in a circle having a diameter of 1.5 cm about the bacterial culture inoculation point in agroinfiltration, through image analysis.

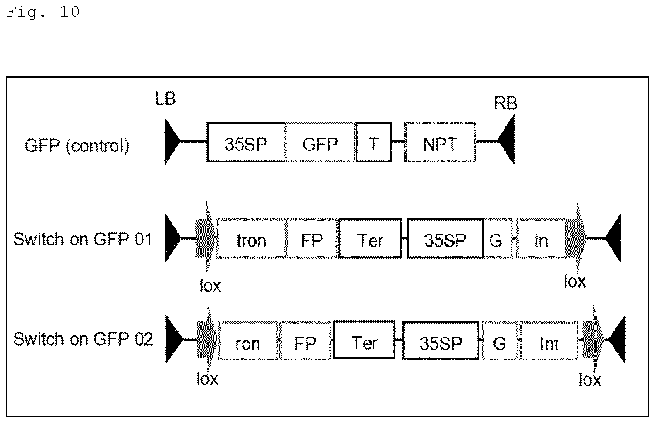

[Fig. 10] Fig. 10 is a schematic diagram of vectors prepared for verifying the activation of the DNA construct of the present invention.

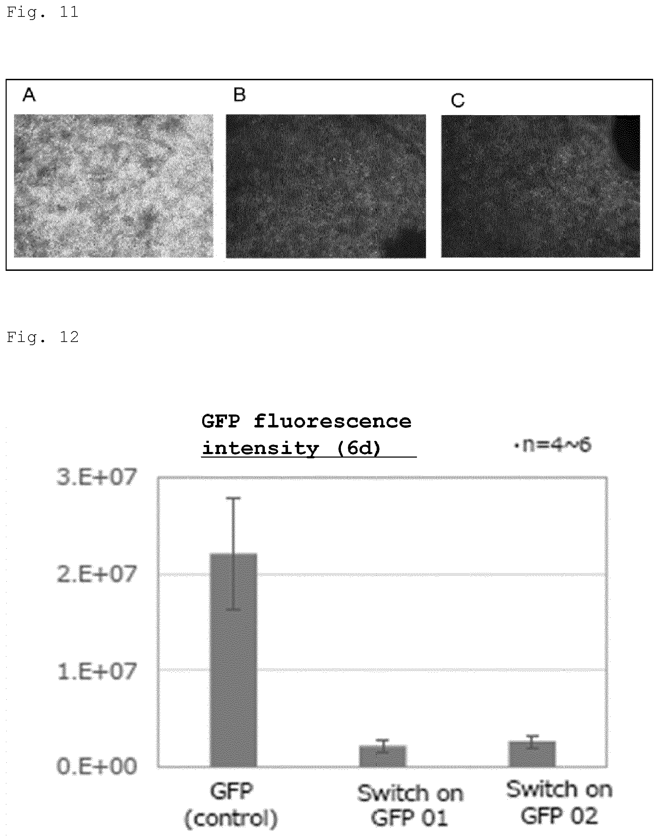

[Fig. 11] Fig. 11 is pictures showing the results of observing leaf pieces of Nicotiana benthamiana into which the vectors shown in Fig. 10 were introduced through agroinfiltration, with fluorescence microscope. More specifically, Fig. 11 shows the results of observing GFP fluorescence of the leaf pieces on day 7 from the agroinfiltration into Nicotiana benthamiana. In the upper stage, A indicates GFP vector-derived GFP fluorescence used as control, B indicates Switch on GFP 01 vector-derived GFP fluorescence, and C indicates Switch on GFP 02 vector-derived GFP fluorescence.
[Fig. 12] Fig. 12 is a graph showing the results of detecting fluorescence intensity in the leaf pieces of Nicotiana benthamiana into which the vectors shown in Fig. 10 were introduced through agroinfiltration. More specifically, Fig. 12 shows the results of quantifying the GFP fluorescence intensity, for each leaf piece on day 6 shown in Fig. 8, in a circle having a diameter of 1.5 cm about the bacterial culture inoculation point in agroinfiltration, through image analysis.

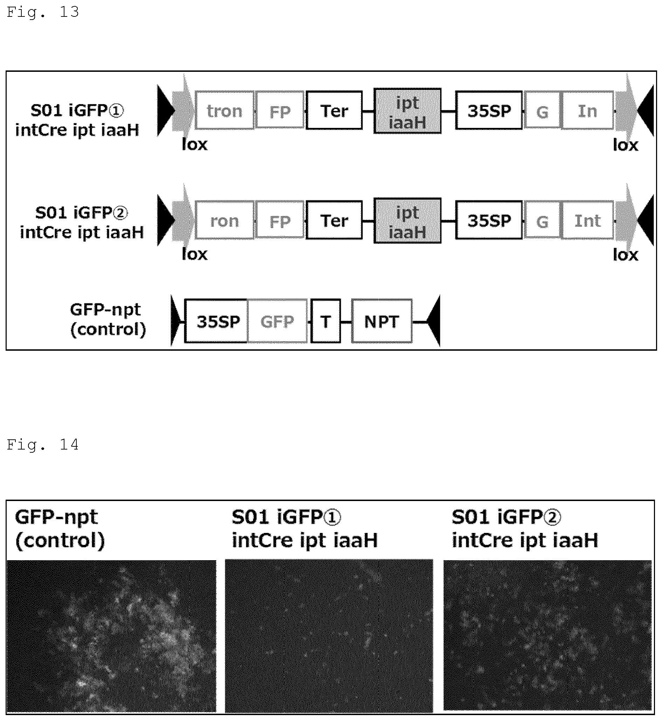

[Fig. 13] Fig. 13 is a schematic diagram of improved vectors prepared for verifying the activation of the DNA construct of the present invention. In the figure, "Ipt" indicates the cytokinin biosynthesis gene and "iaaH" indicates the auxin precursor biosynthesis gene.
[Fig. 14] Fig. 14 is pictures showing the results of observing leaf pieces of Nicotiana benthamiana into which the vectors shown in Fig. 13 were introduced through agroinfiltration, with fluorescence microscope. More specifically, Fig. 14 shows the results of observing GFP fluorescence of the leaf pieces on day 7 from the agroinfiltration into Nicotiana benthamiana. In the upper stage, "GFP-npt (control)" indicates the GFP vector-derived GFP fluorescence used as control and "S01 iGFP (1) intCre ipt iaaH" and "S01 iGFP(2) intCre ipt iaaH" each indicate the improved vector-derived GFP fluorescence.

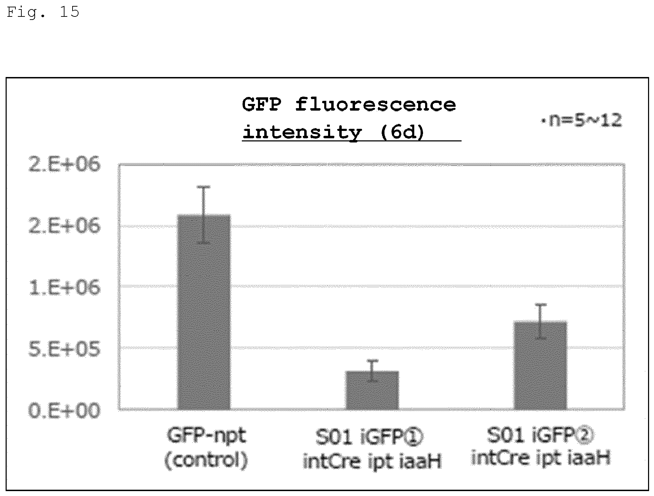

[Fig. 15] Fig. 15 is a graph showing the results of detecting fluorescence intensity in the leaf pieces of Nicotiana benthamiana into which the vectors shown in Fig. 13 were introduced through agroinfiltration. More specifically, Fig. 15 shows the result of quantifying the GFP fluorescence intensity, for the leaf pieces on day 6 shown in Fig. 8, in a circle having a diameter of 1.5 cm about the bacterial culture inoculation point in agroinfiltration, through image analysis.

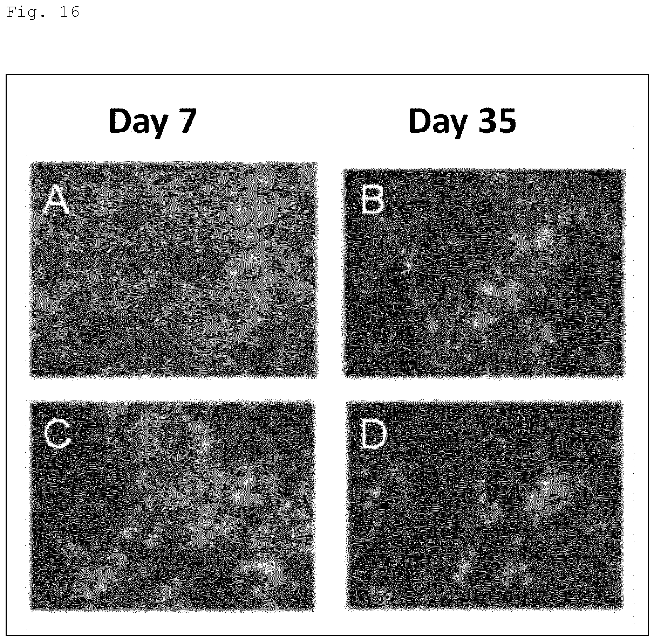

[Fig. 16] Fig. 16 is pictures showing the results of observing the leaf pieces of Nicotiana benthamiana into which the vectors shown in Fig. 13 were introduced through agroinfiltration, with fluorescence microscope. In the figure, A and B show GFP fluorescence respectively on day 7 and day 35 after the infection, derived from the ipt and iaaH-incorporated, improved vector (S01 iGFP (1) intCre ipt iaaH). In the figure, C and D show GFP fluorescence respectively on day 7 and day 35 after the infection, derived from the ipt and iaaH-incorporated, improved vector (S01 iGFP(2) intCre ipt iaaH).

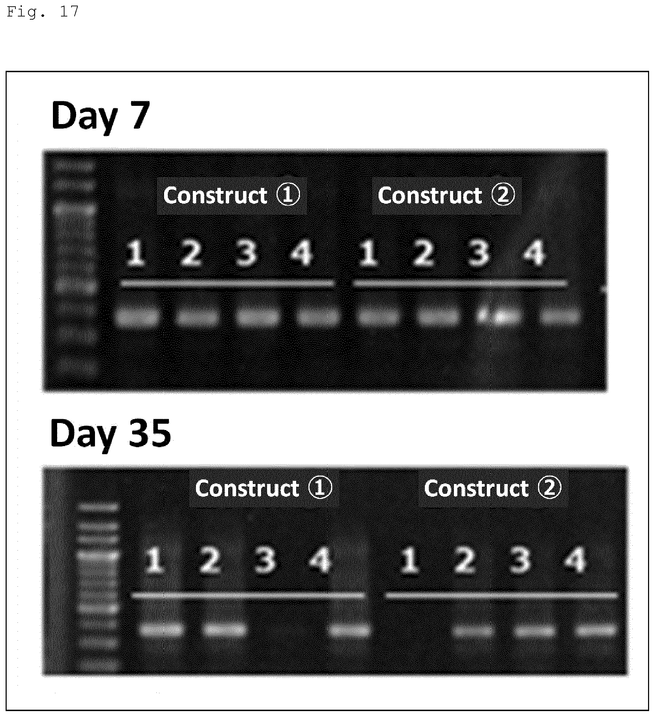

[Fig. 17] Fig. 17 is electrophoresis pictures showing the results of analyzing the leaf pieces of Nicotiana benthamiana into which the vectors shown in Fig. 13 were introduced through agroinfiltration, with genomic PCR. The upper stage shows the results of analysis on day 7 after the infection of the ipt and iaaH-incorporated, improved vector and the lower stage shows the results of analysis on day 35 after the infection of the ipt and iaaH-incorporated, improved vector.

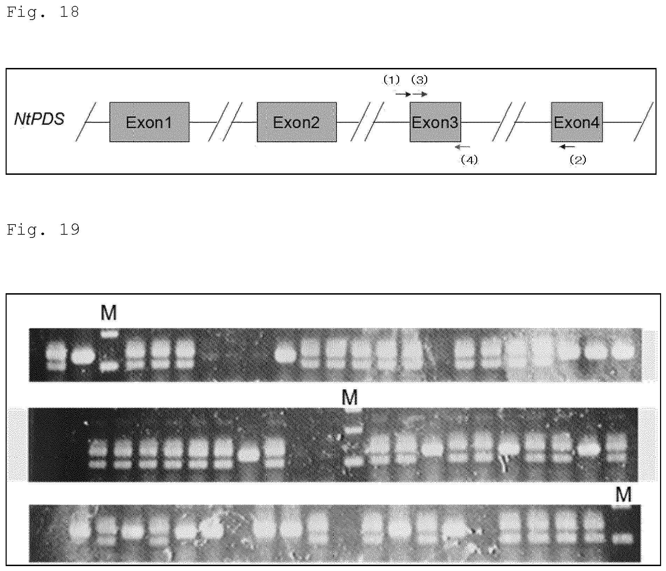

[Fig. 18] Fig. 18 is a schematic diagram showing the configuration of NtPDS gene which was a target gene for genome editing in Examples. As the target sequence for the genome editing, the third exon sequence (PDS3) of PDS, which is a consensus sequence of Arabidopsis theliana and SR1 tobacco, was selected. Specifically, the target sequence corresponds to the third exon portion of PDS. The SgRNA target sequence of PDS3 is GAGGCAAGAGATGTCCTAGGTGG (SEQ ID NO: 21). Note that TGG at the 3' terminal in this sequence is the PAM sequence. In addition, insert used in blunt-end cloning into pUC118 was amplified using the primer sets (1) and (2). In conducting colony PCR, the primer sets (3) and (4) were used.
[Fig. 19] Fig. 19 is electrophoresis pictures showing the results of analyzing the PDS3 gene of SR1 WT with colony PCR. Fig. 19 shows the results of conducting colony PCR using Emerald Amp on colonies obtained in the blunt-end cloning into pUC118 described in Examples. In the figure, "M" indicates marker lanes obtained by conducting electrophoresis on 500 bp DNA Ladder.

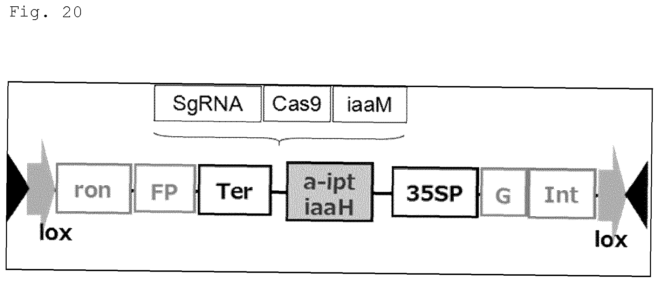

[Fig. 20] Fig. 20 is a schematic diagram showing the configuration of the DNA construct of the present invention, used in verifying the activation of the genome editing system (Cas9 protein and SgRNA). In the figure, "a-ipt" indicates ipt gene having a promoter derived from the agrobacterium pO22 line.

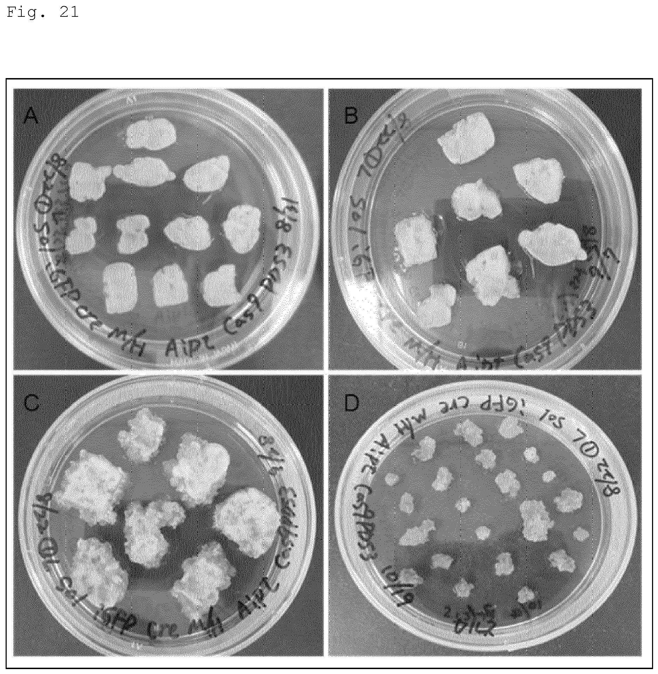

[Fig. 21] Fig. 21 is pictures showing the result of introducing the DNA construct shown in Fig. 20 into leaf pieces of tobacco (SR1) through the agrobacterium method and culturing the leaf pieces. After the infection, the leaf pieces were placed on a selective medium that kills agrobacterium for 2 days, and thereafter were subcultured with a hormone-free medium once a week. In the figure, A indicates the result of observing the leaf pieces of SR1 on day 9 after the infection of agrobacterium, B indicates the result of observing the leaf pieces of SR1 on day 16 after the infection, C indicates the result of observing SR1 on day 40 after the infection, which shows the formation of calli from the leaf pieces. D shows calli separated from the leaf pieces.

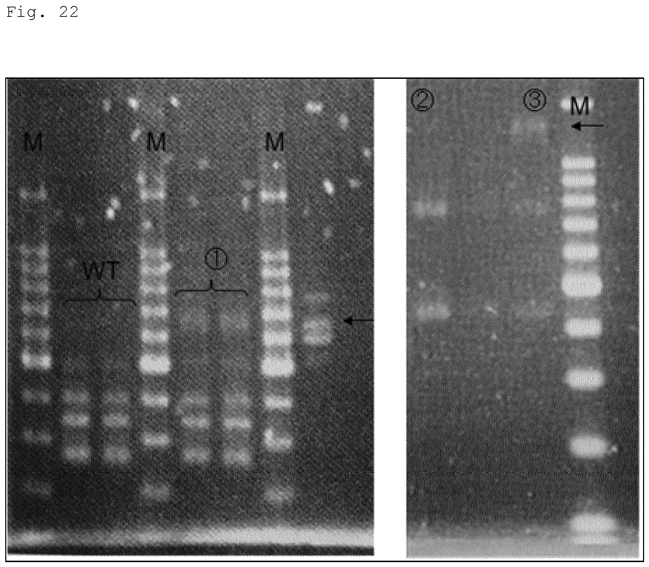

[Fig. 22] Fig. 22 is electrophoresis pictures showing the results of CAPS analysis on calli obtained by introducing the DNA construct shown in Fig. 20 into the leaf pieces of tobacco (SR1) through the agrobacterium method and culturing the leaf pieces . PCR reaction was conducted using the primer set shown in Fig. 18 with the genomic DNA extracted from calli as template, and the amplicons obtained were treated with restriction enzyme BlnI at 37°C for 4 hours. The electrophoresis picture on the left side shows the result of CAPS analysis on the calli on day 39 after the infection. In this analysis, the primers (1) and (2) shown in Fig. 18 were used. In the figure, "WT" indicates a fragment obtained by amplifying PDS3 of SR1, and serves as a rough indication for uncleavage. The electrophoresis picture on the right side shows the result of CAPS analysis on calli on day 45 after the infection. In this analysis, the primers (3) and (4) shown in Fig. 18 were used. "M" in the figure indicates the marker lane showing the result of conducting electrophoresis on 100 bp DNA Ladder. Black arrows indicate positions of uncleaved fragment in Bln1. Note that circle 1 and circle 3 in the figure are samples in which mutagenesis was detected by the CAPS analysis. Circle 2 in the figure a sample in which no mutation was detected by the CAPS analysis.

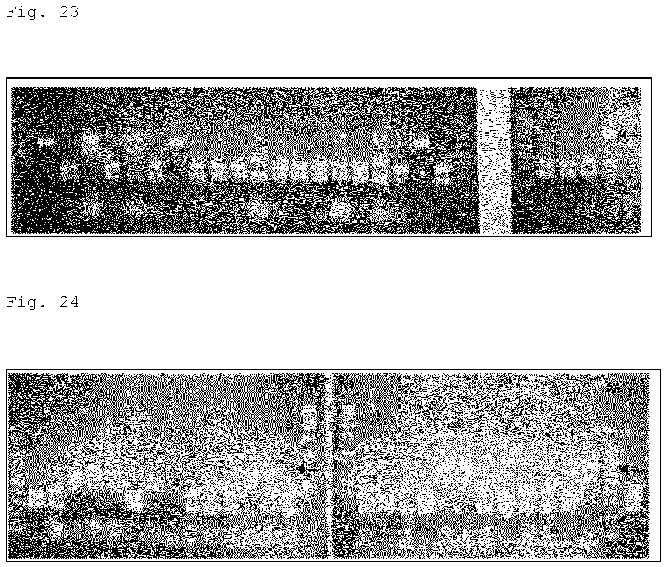

[Fig. 23] Fig. 23 is electrophoresis pictures showing the result of conducting CAPS analysis on calli (on day 39 after the infection) obtained by introducing the DNA construct shown in Fig. 20 into leaf pieces of tobacco (SR1) by the agrobacterium method and culturing the leaf pieces. Blunt-end cloning was conducted on the calli, and colony PCR was conducted on the colonies obtained to amplify PDS3. Thereafter, the samples treated with BlnI at 37°C for 4 hours were subjected to electrophoresis, the results of which are shown. In the figure, "M" indicates lanes obtained by conducting electrophoresis on 100 bp marker, and black arrows indicate the positions of uncleaved fragments.
[Fig. 24] Fig. 24 is electrophoresis pictures showing the results of conducting CAPS analysis on calli (on day 45 after the infection) obtained by introducing the DNA construct shown in Fig. 20 into leaf pieces of tobacco (SR1) by the agrobacterium method and culturing the leaf pieces. Blunt-end cloning was conducted on the calli, and colony PCR was conducted on the colonies obtained to amplify PDS3. Thereafter, the samples treated with BlnI at 37°C for 4 hours were subjected to electrophoresis, the results of which are shown. In the figure, "M" indicates lanes obtained by conducting electrophoresis on 100 bp marker, and black arrows indicate the positions of uncleaved fragments.

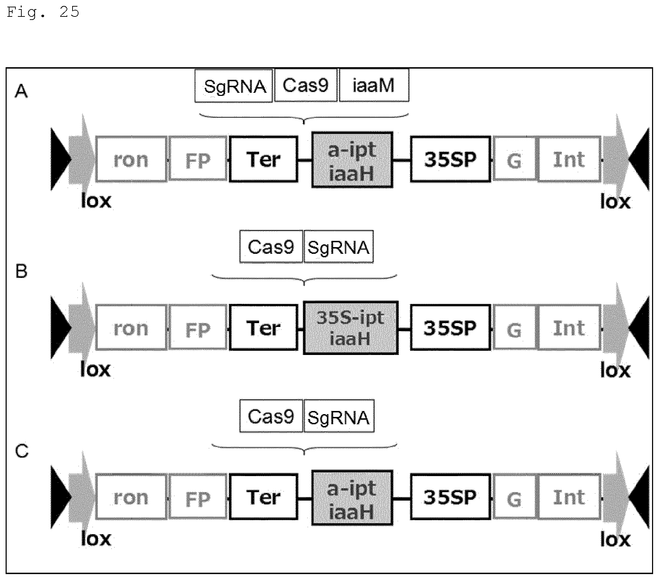

[Fig. 25] Fig. 25 is a schematic diagram showing the configurations of the DNA construct of the present invention, used in morphology selection and mutagenesis. In the figure, "35S-ipt" indicates the ipt gene obtained by changing the promoter from the promoter derived from agrobacterium pO22 line to 35S promoter.

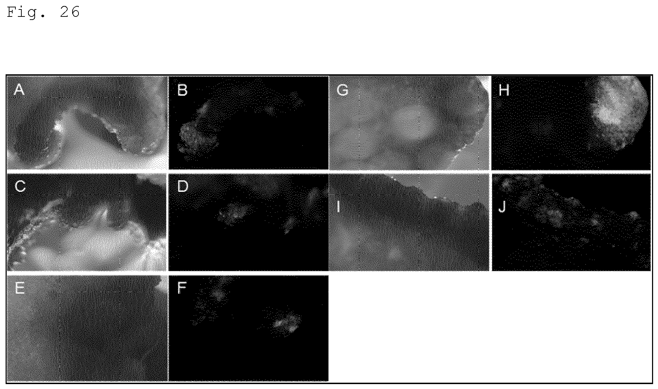

[Fig. 26] Fig. 26 is pictures showing the results of observing calli obtained by introducing the DNA constructs shown in Fig. 25. The DNA constructs were introduced into SR1 by the agrobacterium method. Callus formation in the peripheral portion was observed 30 days after the infection (see A, C, E, G, and I in the figure) . As a result of observing the GFP fluorescence of leaf pieces in which calli were formed, GFP fluorescent calli was observed in 5 leaf pieces among 18 leaf pieces (see B, D, F, H, and J in the figure). A to D in the figure show calli obtained by introducing the construct A shown in Fig. 25 and growing the leaf pieces in MS medium. E to H in the figure show calli obtained by introducing the construct B shown in Fig. 25 and growing the leaf pieces in NAM-containing MS medium. I and J in the figure show calli obtained by introducing the construct C shown in Fig. 25 and growing the leaf pieces in MS medium.

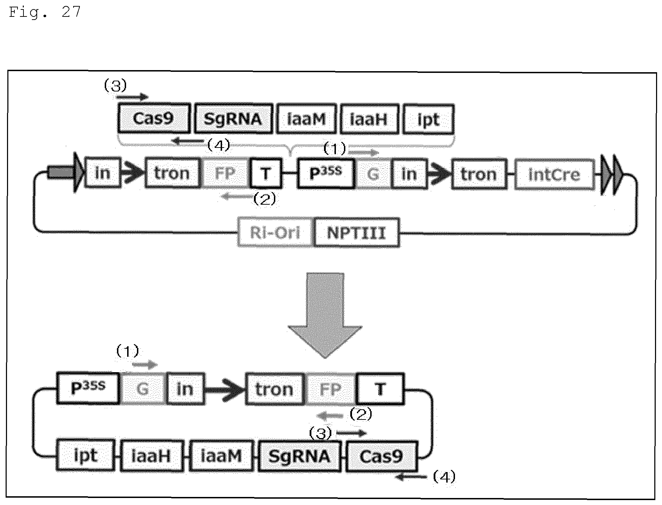

[Fig. 27] Fig. 27 is a schematic diagram showing the DNA construct of the present invention, and the positional relation between cyclic DNA generated from the DNA construct and the primer used for verifying the behavior of the cyclic DNA. The primers (1) and (2) are a primer set for amplifying the coding region fragment of gfp, which are designed such that the PCR reaction proceeds once the cleavage to cyclic DNA occurs, and were used for detecting cyclic DNA. The primers (3) and (4) are a primer set for amplifying the coding region fragment of Cas9, and were used to confirming the presence of Cas9. The primers (3) and (4) were used to confirm the cleavage to cyclic DNA from the length of the amplified band.

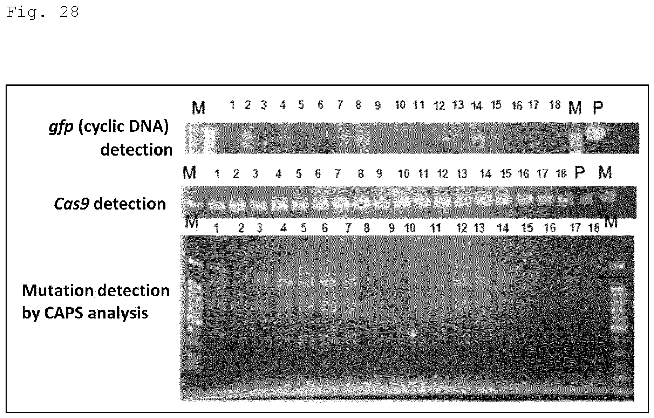

[Fig. 28] Fig. 28 is electrophoresis pictures showing the results of conducting genomic PCR analysis and CAPS analysis on calli obtained by introducing the DNA constructs shown in Fig. 25. The gfp gene (cyclic DNA) and Cas9 gene were detected by extracting genomic DNA from calli on day 30 after the infection and conducting genomic PCR using the primer sets shown in Fig. 27. In addition, CAPS analysis was conducted by the method described in Fig. 22 to detect mutagenesis in the target gene. The lanes 1 to 6 show the calli obtained by introducing the construct A shown in Fig. 25 and growing in MS medium. The lanes 7 and 8 show calli obtained by introducing the construct B shown in Fig. 25 and growing in MS medium. The lanes 9 to 13 show calli obtained by introducing the construct B shown in Fig. 25 and growing in NAM-containing MS medium. The lanes 14 to 18 show calli obtained by introducing the construct C shown in Fig. 25 and growing in MS medium. In addition, "M" in the figure indicates marker lanes obtained by conducting electrophoresis on DNA Ladder 500 bp. Black arrow indicates the position of uncleaved fragment in CAPS analysis.

### [Description of Embodiments]

### (DNA Construct)

A DNA construct for expressing a genome editing system in a plant cell of the present invention comprises: a cleaving target region flanked by a first recombinase recognition site located on a 5' side and a second recombinase recognition site located on a 3' side, wherein
the first recombinase recognition site and the second recombinase recognition site are in the same direction,
a gene encoding a site-specific recombinase that specifically recognizes the first and second recombinase recognition sites is arranged outside of at least one side of the cleaving target region,
the cleaving target region contains: a gene encoding the genome editing system; and a marker gene divided into a 5' region and a 3' region,
the 3' region of the marker gene is adjacent to a 3' terminal of the first recombinase recognition site, the 5' region of the marker gene is adjacent to a 5' terminal of the second recombinase recognition site, and the gene encoding the genome editing system is arranged between the 3' region and the 5' region of the marker gene.

With this configuration, when the DNA construct of the present invention is introduced into a plant cell, the site-specific recombinase expresses to cleave out and circularize the cleaving target region, causing the 5' region and the 3' region to bind via the recombinase recognition site, which allows the marker gene to express. In addition, the circularized region stably exists in the plant cell without being incorporated into the genome of the cell for a certain period of time and allows the genome editing system to be encoded to express. For this reason, it is possible to efficiently obtain a genome-edited plant cell.

The "cleaving target region" according to the present invention is such that 2 recombinase recognition sites (the first recombinase recognition site located on the 5' side and the second recombinase recognition site located on the 3' side), which are specifically recognized by the site-specific recombinase, are arranged in the same direction at the terminals of the cleaving target region in order to be cleaved out and circularized by the site-specific recombinase.
in the present invention, the "site-specific recombinase" only has to be an enzyme that recognizes specific sequences (recombinase recognition sites) and causes recombination between the sequences, and is not particularly limited but includes, for example, Cre recombinase, recombinase R derived from soy sauce yeast (Zygosaccharomyces rouxii), FLP recombinase, γδ resolvase, Tn3 resolvase, ΦC31 integrase, Bxb1-integrase, R4 integrase, and λ integrase. In addition, the "recombinase recognition sites" only have to be sequences that can be specifically recognized by the respective enzymes, and include, for example, loxP sequence for Cre recombinase, Rs sequence for recombinase R, and FRT sequence for FLP recombinase.

The "marker gene" contained in the cleaving target region only has to be capable of allowing the marker protein encoded by the gene to express in a plant cell and be detected, and includes, for example, fluorescent protein genes, luminescent enzyme genes, chromogenic enzyme genes, and drug resistance genes.

The fluorescent protein genes specifically include, for example, GFP (green fluorescent protein) gene, YFP (yellow fluorescent protein) gene, RFP (red fluorescent protein) gene, and aequorin gene. The luminescent enzyme genes specifically include, for example, luciferase gene. The chromogenic enzyme genes specifically include, for example, β glucuronidase (GUS) gene, β galactosidase gene, alkaline phosphatase gene, and SEAP gene. The drug resistance genes specifically include, for example, kanamycin resistance (NPTII) gene, hygromycin resistance (HPT) gene, tetracycline resistance gene, ampicillin resistance gene, spectinomycin resistance gene, chloramphenicol resistance gene, neomycin resistance gene, herbicide resistance genes (bialaphos resistance gene, glyphosate resistance gene (EPSPS), and sulfonylurea resistance gene (ALS)).

As shown in Examples described later, the "marker gene" is contained, while divided into the 5' region and the 3' region, in the cleaving target region so that the circularization of the cleaving target region and the circularized DNA can be monitored. Moreover, the marker gene is arranged in the cleaving target region such that the 3' region is adjacent to the 3' terminal of the first recombinase recognition site and the 5' region is adjacent to the 5' terminal of the second recombinase recognition site. With this arrangement, when recombination occurs between the first recombinase recognition site and the second recombinase recognition site due to the action of the aforementioned site-specific recombinase, the 5' region and the 3' region of the marker gene bind to be reconstructed.

The division of the marker gene is not particularly limited as long as the division can express a functional protein, and may be, for example, in exon of the marker gene or in intron of the marker gene. However, from the viewpoints that even when modification occurs in the sequence, the effect of the modification is small, and that the division is easily designed, it is preferable that the marker gene is divided into the 5' region and the 3' region in intron.

In addition, it is satisfactory that at least the gene encoding the genome editing system is arranged between the 3' region and the 5' region of the marker gene. However, if the terminator region contained in the 3' region of the marker gene and the promoter region contained in the 5' region of the marker gene come too close, this tends to impose a negative effect on the expression of the marker protein. For this reason, the distance between the 3' region and the 5' region is preferably 2000 bases or more, and more preferably 4000 bases or more.

In the cleaving target region according to the present invention, a gene encoding the genome editing system is arranged between the 3' region and the 5' region of the marker gene. In the present invention, the "genome editing system" is not particularly limited as long as the genome editing system is an artificial restriction enzyme system that can cause modification in any desired site on a genomic DNA. The artificial restriction enzyme system includes, for example, the CRISPR/Cas system, the TALEN system, the ZFN system, and the PPR system.

The CRISPR/Cas system uses Cas protein, which is a nuclease (RGN; RNA-guided nuclease) , and a guide RNA (g RNA, Single-guide RNA (SgRNA) ) . By introducing the system into a cell, it is possible to cause the guide RNA to bind to the target site, and cleave DNA with Cas protein induced into the binding site (for example, CRISPR-Cas9 (United States Patent No. 8697359, International Publication NO. WO2013/176772), CRISPR-Cpf1 (Zetsche B. et al., Cell, 163(3): 759-71, (2015)).

In addition, an artificial enzyme complex obtained by adding a deaminase, which is a deamination enzyme, to what is obtained by removing the nuclease activity from the CRISPR/Cas system can also be used as the genome editing system according to the present invention (Target-AID (K. Nishida et al., Targeted nucleotide editing using hybrid prokaryotic and vertebrate adaptive immune systems, Science, DOI: 10.1126/science.aaf8729, (2016))).

The TALEN system uses an artificial nuclease (TALEN) containing a transcription activator-like (TAL) effector DNA-binding domain in addition to a DNA cleavage domain (for example, FokI domain) (for example, United States Patent No. 8470973, United States Patent No. 8586363) . By introducing the system into a cell, it is possible to cause TALEN to bind to the target site via the DNA-binding domain to cleave DNA there. The DNA-binding domain to bind to the target site can be designed in accordance with a publicly-known scheme (for example, Zhang, Feng et. al. (2011) Nature Biotechnology 29 (2)).

The ZFN system uses an artificial nuclease (ZFN) containing a nucleic acid cleavage domain conjugated to a DNA-binding domain containing a zinc finger array (for example, United States Patent Nos. 6265196, 8524500, and 7888121, and European Patent No. 1720995) . By introducing the system into a cell, it is possible to cause ZFN to bind to the target site via the DNA-binding domain to cleave DNA there. The DNA-binding domain to bind to the target site can be designed in accordance with a publicly-known scheme.

As the PPR system, for example, PPR (pentatricopeptide repeat) to which a nuclease domain is fused can be used (Nakamura et al., Plant Cell Physiol 53:1171-1179(2012)).

Among these genome editing systems, CRISPR/Cas system is preferable from the viewpoint that the CRISPR/Cas system allows the target site to be more freely selected because of using a nucleic acid (guide RNA) for recognizing the target site, and also is easily prepared. Note that the length of the guide RNA used in the CRISPR/Cas system is at least 15, 16, 17, 18, 19, or 20 bases. The upper limit for the number of bases is preferably 30 or less, more preferably 25 or less, further preferably 22 or less, and most preferably 20 or less. The Cas protein may contain one or more nuclear localization signals (NLSs). In addition, the Cas protein is preferably type II CRISPR enzyme, and is for example, Cas9 protein. The Cas9 protein is Streptococcus pneumoniae (S. pneumoniae), Streptococcus pyogenes (S. pyogenes) Cas9 or S. thermophilus Cas9, may contain a spontaneous mutation Cas9 derived from these living organisms, and may be Cas9 homolog or ortholog.

In the cleaving target region according to the present invention, another exogenous gene may further contained between the 3' region and the 5' region of the marker gene, besides the gene encoding the genome editing system. The "exogenous gene" is not particularly limited, and includes, for example, phytohormone biosynthetic genes, replication promoter genes, and cell death suppressor genes.

The phytohormone biosynthetic genes specifically include ipt gene, iaaM gene, iaaH gene, rol (ro1A, rolB, rolC) genes, and the like. Genes that promote the formation of adventitious embryos include WSU gene, BBM gene, AGL15 gene, Lec1 gene, and the like. The replication promoter genes specifically include APS gene, ct-ars1 gene, and the like. The cell death suppressor gene specifically include ACCd gene, Bax-I gene, NaGH gene, and the like.

In the "DNA construct" of the present invention, besides the aforementioned cleaving target region, only a gene encoding the site-specific recombinase has to be arranged outside at least one side of the cleaving target region; however the DNA construct may further contain another gene. The "another gene" is not particularly limited, and includes, for example, replication origins (mutant-type replication origin (Ri-ori) derived from Ri plasmid, and the like) for allowing the DNA construct of the present invention to be replicated in a host (for example, E. coli) other than plant cell and marker genes (NPTIII gene and the like) functioning in the host.

Note that in the present invention, not only the coding region of protein or RNA, but also a region that controls the expression thereof (promoter, enhancer, insulator, terminator, poly-A additional signal, and the like) may be added to each gene contained in the DNA construct as appropriate. The "promoter" only has to be DNA that can function in a plant cell to constitutively express or induce expression in a specific tissue or in a specific developmental stage of a plant. The "promoter" includes, for example, cauliflower mosaic virus (CaMV) 35S promoter, E112-35S omega promoter, nopaline synhase gene promoter (Pnos) , ubiquitin promoter derived from corn, actin promoter derived from rice, PR protein promoter derived from tobacco, ADH promoter, and Rubisco promoter. The "terminator" only has to be a sequence that can terminate transcription by the promoter and has poly-A additional signal, and includes, for example, terminator of heat shock protein (HSP) gene, terminator of nopaline synhase (NOS) gene, terminator of octopine synthase (OCS) gene, and CaMV 35S terminator.

In addition, in the "DNA construct" of the present invention, when recombination is caused by the site-specific recombinase, the range of the recombination is not only between the recombinase recognition sites but also extends to the surrounding regions. For this reason, it is preferable that the 3' terminal site of the 5' region of the marker gene be further arranged at the 5' terminal of the first recombinase recognition site and the 5' terminal site of the 3' region of the marker gene be further arranged at the 3' terminal of the second recombinase recognition site. In this case, the length of the 3' terminal site of the 5' region and the length of the 5' terminal site of the 3' region is not particularly limited, but is preferably 10 bases or more, more preferably 50 bases or more, further preferably 80 bases or more, and more preferably 100 bases or more.

The form of the "DNA construct" of the present invention is not particularly limited as long as the DNA construct allows the arranged gene to express in the plant cell to which the DNA construct is introduced, and includes, for example, pUCs (pUC57, pUC18, pUC19, pUC9, and the like) , pALs (pAL51, pAL156, and the like), pBIs (pBI121, pBI101, pBI221, pBI2113, pBI101.2, and the like), pPZPs, pSMAs, intermediate vectors (pLGV23Neo, pNCAT, and the like), cauliflower mosaic virus (CaMV), bean golden mosaic virus (BGMV), and tobacco mosaic virus (TMV).

In the case where the DNA construct of the present invention is introduced into a plant cell by the agrobacterium method as shown in Examples described below, it is desirable that the construct further comprises: a right boundary sequence (RB) and a left boundary sequence (LB) that are derived from an agrobacterium T-DNA sequence . Note that in this case, the cleaving target region and the gene encoding the site-specific recombinase are arranged in a transfer region flanked by the RB and the LB.

Moreover, from the viewpoint of enhancing the formation of the transfer region in agrobacterium and promoting the transfer of the region into a plant cell, it is preferable that an overdrive (OD) sequence be adjacent to the 5' terminal of the RB. In addition, a plurality of LBs (for example, 2 LBs) may be arranged. Moreover, a telomere sequence may be adjacent to each of the 3' terminal of the RB and the 5' terminal of the LB (see Chiurazzi M. et al., Plant Molecular Biology, 1994, 26, 923-934, and Teo CH. et al, The Plant Journal, 2011, 68, 28-39).

In addition, the DNA construct of the present invention can be fabricated by a person skilled in the art using a publicly-known method as appropriate. For example, the DNA construct can be fabricated by cleaving a purified gene cassette with an appropriate restriction enzyme, and connecting the cleaved part to a restriction enzyme site, a multiple cloning site, or the like of an appropriate vector, serving as a backbone as shown in Examples described below. In addition, the DNA construct of the present invention can also be fabricated using methods such as In-Fusion cloning, TA cloning, double cross-over. Moreover, in order to allow the translation products to efficiently express in plant cells into which the DNA construct of the present invention is introduced, the nucleic acid sequence of the gene encoded in the DNA construct of the present invention may be optimized into a codon suitable for the cell.

### (Method for Producing Genome-edited Plant Cell)

A method for producing a genome-edited plant cell of the present invention is a method comprising the steps of:
(1) introducing the above-described DNA construct of the present invention into plant cells; and
(2) selecting a cell in which the DNA construct has been introduced and circularization of the cleaving target region has occurred from cells obtained in the step (1) using expression of the marker gene as an indicator.

As shown in Examples described below, this method makes it possible to select cells in which circularization of the cleaving target region has occurred, that is, cells in which the gene encoding the genome editing system and the like contained in the region have not been incorporated into the plant genome, and thus to obtain plant cells in which only the target gene has been modified.

The "plant" to be subjected to the genome editing in the method of the present invention is a seed plant including angiosperms and gymnosperms, and angiosperms include monocots and dicots. The monocots include, for example, Gramineae plants, Liliaceae plants, Musaceae plants, Bromeliaceae plants, and Orchidaceae plants. The dicots include, for example, Brassicaceae plants, Fabaceae plants, Solanaceae plants, Cucurbitaceae plants, Convolvulaceae plants, Rosaceae plants, Moraceae plants, Malvaceaeplants, Asteraceae plants, Amaranthaceae plants, and Polygonaceae plants. In addition, the gymnosperms include, for example, Pinus, Cryptomeria japonica, Ginkgo biloba, and Cycas revoluta.

The "plant cells" into which the DNA construct of the present invention is introduced include, besides cells in the aforementioned plants, culture cells derived from the plants. Moreover, the plant cells include plant cells in various forms, for example, leaf sections, seeds, stem apexes of embryos of seeds, stem apexes of plumules of tubers, suspended culture cells, protoplasts, calli, immature embryos, pollens, and the like.

The "introducing" the DNA construct of the present invention into plant cells can be conducted by a person skilled in the art using a publicly-known method as appropriate, and such a method includes, for example, agrobacterium methods such as the agroinfiltration, particle bombardment methods such as the iPB (in planta Particle Bombardment), the electroporation method, the PEG-calcium phosphate method, the liposome method, the microinj ection method, the whisker method, the plasma method, and the laser injection method. Note that the iPB method is disclosed in, for example, Japanese Unexamined Patent Application Publication No. 2017-205104 and Japanese Unexamined Patent Application Publication No. 2017-205103.

From the plant cells into which the DNA construct of the present invention has been inserted, cells in which the DNA construct has been introduced and circularization of the cleaving target region has occurred can be selected using expression of the marker gene as an indicator. The "selecting" can be conducted by a person skilled in the art as appropriate depending on the type of the marker gene. For example, in the case where the marker gene is a fluorescent protein gene, cells can be selected by detecting expression of the fluorescent protein gene with a fluorescence microscope. In addition, cells can also be selected with a cell sorter or the like by utilizing a difference in fluorescence intensity. In the case of using a luminescent enzyme gene, cells can be selected by adding a luminescent substrate to cells and measuring the amounts of luminescence with a luminometer to detect the expression. In the case of using a chromogenic enzyme gene, cells can be selected by adding a chromogenic substrate to cells and observing whether the cells are colored to detect the expression. In the case of using a drug resistance gene, cells can be selected by culturing cells with a medium (selective medium) containing the corresponding drug.

In addition, in the present invention, in the case where a DNA construct that also contains a phytohormone biosynthetic gene is used as the exogenous gene, an aspect of a method for producing a genome-edited plant as described below is possible.

A method for producing a genome-edited plant, the method comprising the steps of:
(1) introducing a DNA construct of the present invention that also contains a phytohormone biosynthetic gene into plant cells;
(2) selecting a cell in which the DNA construct has been introduced and circularization of the cleaving target region has occurred from cells obtained in the step (1) using expression of the marker gene as an indicator; and
(3) culturing the cell obtained in the step (2) to generate plant tissues, and selecting a plant tissue in which morphological change has occurred, using the morphological change in the plant tissues attributable to expression of the phytohormone biosynthetic gene on the DNA construct as an indicator.

The steps (1) and (2) in this method are the same as those described above. In the step (3), by using morphological change in the plant tissues attributable to expression of the phytohormone biosynthetic gene as an indicator, it is possible to select a plant tissue in which the DNA construct of the present invention has been introduced and circularization of the cleaving target region has occurred. Note that the method for culturing plant cells to eventually generate plant tissues is not particularly limited, and can be conducted by a person skilled in the art as appropriate using publicly-known medium and the like as shown in Examples described later.

In addition, the "selecting" in the step (3) can also be conducted by a person skilled in the art as appropriate depending on the type of the phytohormone biosynthetic gene. For example, in the case where the phytohormone biosynthetic gene is ipt gene, a plant tissue can be selected using adventitious bud formation or callus formation as an indicator. In the case where the phytohormone biosynthetic gene is iaaM gene and iaaH gene, a plant tissue can be selected using adventitious root formation or callus formation as an indicator. In the case where the phytohormone biosynthetic gene is ipt gene, iaaM gene, and iaaH gene, a plant tissue can be selected using adventitious root formation or callus formation as an indicator. In the case where the phytohormone biosynthetic gene is rol gene, a plant tissue can be selected using hairy root formation or callus formation as an indicator. In the case where the phytohormone biosynthetic gene is AGL15 gene, a plant tissue can be selected using adventitious embryo formation as an indicator. In the case where the phytohormone biosynthetic gene is BBM gene, WSU gene, or Lec1 gene, a plant tissue can be selected using callus formation as an indicator.

In addition, the confirmation that the sequence of the target gene has been modified in plant cells or the like thus selected and obtained can be made by a person skilled in the art appropriately using a publicly-known method. The confirmation can be made, for example, using the sequence (sequencing) method, the PCR method, the CAPS method, the RFLP method, the Southern blotting method, or the like. In addition, whether genome editing has been made in the target gene can also be confirmed by analyzing the presence or absence, the amount, the molecular weight, and the like of RNA expressed from the gene. The confirmation can also be made, for example, using the RT-PCR method or the Northern blotting method. Moreover, the confirmation can also be made by analyzing the presence or absence, the amount, the molecular weight, and the like of protein expressed from the target gene. The confirmation can be made, for example, using the Western blotting method, the immunostaining, the ELISA method, the RIA, or the dot-immunobinding assay.

In addition, for plants, it has been long known that the somatic cells of plants have totipotency, and methods for regenerating plants from plant cells and plant tissues (calli and the like) have been developed for various plants (for example, see Tabei Y. Ed., "Protocols of Transformation [Plants], Kagaku-Dojin Publishing Company, INC, published on September 20, 2012) . Hence, for example, it is possible to obtain a plant in which the sequence of the target gene has been modified by regenerating the plant from the plant cell or the plant tissue obtained by the method of the present invention.

### [Examples]

The present invention will be described in more detail below based on Examples; however, the present invention is not limited to Examples described below. In addition, Examples were conducted using experimental materials and methods described below. Note that reagents with no manufacturer's name below are those manufactured by Wako Pure Chemical.

### (Preparation and Cultivation of Plants)

Nicotiana benthamiana was used for transient assay. Nicotiana benthamiana does not have immune systems against viruses, and accordingly is used in infection experiments such as agroinfiltration.

SR1 of tobacco (N. tabacum) was used in infection experiment with the immersion method. The genome of SR1 is amphidiploid (2n = 24) and is derived from ancestors of N. sylvestris (2n = 12: SS) and N. tometosiformis (2n = 12: TT) . The SR1 seeds used in these Examples was provided by JT (Japan Tobacco Inc.).

First, 1 ml of 70% ethanol was added to SR1 seeds and centrifuged at 6000 rpm, and the supernatant was discarded. Then, 1 ml of a sterile solution (10-fold diluted Haiter) was added, followed by agitating for 10 minutes. Thereafter, the supernatant was discarded. In the clean bench, 1 ml of sterile water was added, followed by agitating. Thereafter, the supernatant was discarded. This operation was repeated 3 times. While floating seeds were discarded, the seeds were inoculated into agar medium and left to stand for 2 to 3 weeks in a high-temperature room with full lighting. The seeds were transferred into 200 ml conical beakers in order from those started sprouting, and those growing larger were transferred into jam bottles. The leaves of plants about 2 months later were used in the infection experiments.

### (Media and Antibiotics Used)

The LB medium used was prepared by diluting 10 g of Bacto Tryptone, 5 g of Bacto Yeast Extract, and 10 g of NaCl up to 1000 ml with DW, followed by adjusting to pH 7.2, and thereafter sterilizing in an autoclave (121°C, 20 minutes) . In the case of agar medium, the total amount was prepared into 200 ml, and after pH adjustment, 3 g of Bacto Ager was added, followed by sterilizing in an autoclave (121°C, 20 minutes). In the case where an antibiotic was added to the agar medium, the antibiotic was added immediately before the agar medium was spread on Petri dishes.

The MS medium was prepared by diluting one pack of Murashige and Skoog Plant Salt Mixture (manufactured by Nihon Pharmaceutical Co., Ltd.), MURASHIGE&SKOOG MODIFIED VITAMIN SOLUTION (1000×), 1 ml of MP Biomedicals, and 30 g of sucrose up to 1000 ml with DW, and adjusting the dilution to pH 5.8 with 1 M potassium hydroxide, and then separating the resultant into small packets of 200 ml, to each of which 0.6 g of gellan gum was added, followed by sterilizing with an autoclave (121°C, 20 minutes).

In the case where an antibiotic or acetosyringone was added, the antibiotic or acetosyringone was added immediately before the media were spread on Petri dishes. The antibiotics were used after concentrations of ampicillin, kanamycin, streptomycin, carbenicillin, and cefotaxime were adjusted to 100 mg/l, 50 mg/l, 20 mg/l, 100 mg/l, and 200 mg/l, respectively. The concentration of acetosyringone used was adjusted to 50 mg/l.

In the case where plant hormones were added, the plant hormones were added before pH adjustment such that concentrations became as described below. Regeneration medium: naphthaleneacetic acid (NAA) 0.1 mg/l, 6-benzyladenine (BA) 1.0 mg/l
NAM medium: 1-naphthylacetamide (NAM) 1.0 mg/l.

### (Agroinfiltration)

Single colonies were taken with LB medium (25 mg/L of rifampicin and 50 mg/L of kanamycin) , and were cultured in 2 to 3 ml of liquid LB medium at 28°C, 150 to 200 rpm for 18 hours. LB medium to which 200 µM of acetosyringone was added were added to the cultured agrobacterium culture, followed by culturing at 28°C, 150 to 200 rpm for 24 hours. The culture solutions were centrifuged at 3000 g for 10 minutes to be harvested, followed by resuspending into an infiltration buffer in an amount equivalent to that of the culture solutions. The suspensions were again centrifuged at 3000 g for 10 minutes to be harvested, followed by resuspending into an infiltration buffer in an amount half of that of the culture solution. OD was adjusted to 0.3, and acetosyringone was added to the solution up to 200 µM. The infiltration solution was placed in a syringe and was infused from the back sides of leaves of Nicotiana benthamiana.

### (Transformation)

Transformation into E. coli (JM101) and agrobacterium (LBA4504) was conducted using the heat shock method. First, 1 ng of DNA was added to competent cells, which were then placed on ice for 20 minutes. The cells were heat-shocked using heat block at 42°C for 30 seconds and immediately stuck on ice. Thereafter, liquid LB medium in an amount 5 times the competent cells was added, followed by incubation at 37°C for 1 hour for E. coli and at 28°C for 2 hours for agrobacterium. After the incubation, the bacterial cultures were sprinkled on LB medium containing appropriate antibiotics and spread with bacteria spreader, followed by incubation at 37°C for about 12 hours for E. coli and at 28°C for 48 hours for agrobacterium to obtain colonies.

### (Infection Experiment to SR1 Tobacco by Immersion Method)

agrobacterium into which a vector had been transformed was shaken and cultured at 28°C for 48 hours in LB medium to which kanamycin was added to obtain infected bacterial cultures. Sufficiently grown leaves were cut out of sterile seedlings while avoiding thick veins into leaf pieces having approximately 1 cm square in Petri dishes in which sterile water was placed. The leaf pieces were placed in 50 ml Falcon tube in which 27 ml of sterile water was placed, and 3 ml of the infected bacterial culture was added thereto, followed by sufficient agitation. The leaf pieces were placed in the infected solution such that the entire leaf pieces were immersed in the infected solution for 10 minutes . The leaf pieces were taken out on sterile KIMTOWEL to suck and remove excess bacterial culture. The leaf pieces were planted into MS regeneration medium to which acetosyringone had been added, and cocultured at 28°C for 48 hours under complete dark. The cocultured leaf pieces were planted on MS medium to which carbenicillin and cefotaxime were added such that 16 to 25 pieces were planted per Petri dish, followed by culturing for 1 week under complete dark condition. After 1 week later, culturing was conducted at 25°C under 16 h light/8 h dark condition. Thereafter, the medium was replaced to subculture every week.

### (Agarose Electrophoresis)

In agarose electrophoresis of DNA, Tris-Acetate-EDTA (TAE) electrophoresis buffer (50×) (manufactured by Cosmo Bio Co., Ltd.) was 50-fold diluted with DW to obtain an electrophoresis buffer. As agarose gel, 1% agarose gel prepared by adding and heating and dissolving 1% of Agarose L03 (manufactured by Takara Bio Inc.) to this buffer, and solidifying the resultant in a gel tray was used.

### (Gel Extraction)

After the agarose electrophoresis, DNA was extracted from the gel using FastGene Gel/PCR Extraction Kit (manufactured by Nippon Genetics Co., Ltd.) in accordance with the attached instruction.

### (Restriction Enzyme Treatment)

The restriction enzyme treatment was conducted every time using an enzyme manufactured by Takara Bio Inc. in accordance with the attached instruction.

### (In Fusion Reaction)

The in fusion reaction is a reaction that can bind DNAs in the case where two DNA terminals have 15 bp homologous sequence. Cloning with in fusion reaction was conducted by conducting PCR using PrimeSTAR Max (Manufactured by Takara Bio Inc.) in accordance with the attached instruction, and reacting the PCR product and a vector linearized by restriction enzyme treatment using In Fusion HD Cloning Kit (Manufactured by Takara Bio Inc.) in accordance with the attached instruction. The vector circularized by the in fusion reaction was transformed into E. coli, and the vector was confirmed with colony PCR.

The basic programs of PCR using Prime STAR MAX were as described below.

**[Table 1]**

| Temperature | Time | Cycle count |
|---|---|---|
| 94°C | 2 min | ×1 |
| 98°C | 10 sec | ×30 |
| Tm value | 15 sec | |
| 72°C | 30 sec/lkb | |
| 72°C | 1 min | ×1 |
| 4°C | ∞ | |

### (Colony PCR)

The transformed E. coli colony was taken with the tip of 2 µl chip using Emerald Amp (Manufactured by Takara Bio Inc.) in accordance with the attached instruction to conduct PCR.

The basic programs of PCR using Emerald Amp are shown below.

**[Table 2]**

| Temperature | Time | Cycle count |
|---|---|---|
| 94°C | 2 min | ×1 |
| 98°C | 10 sec | ×30 |
| 60°C | 30 sec | |
| 72°C | 1 min/1kb | |
| 72°C | 1 min | ×1 |
| 4°C | ∞ | |

### (Genome Extraction)

The reaction was conducted using Plant DNA Isolation Reagent (Manufactured by Takara Bio Inc.) in accordance with the attached instruction.

### (Genomic PCR)

All the genomes were adjusted to 50 ng/µl as templates. PCR was conducted using Ex-Taq (Manufactured by Takara Bio Inc.) in accordance with the attached instruction.

The basic programs are shown below.

**[Table 3]**

| Temperature | Time | Cycle count |
|---|---|---|
| 98°C | 2 min | ×1 |
| 98°C | 10 sec | ×30 |
| Tm value | 30 sec | |
| 72°C | 1 min/1kb | |
| 72°C 7 2 | 2 min | ×1 |
| 4°C | ∞ | |

### (Blunt-end Cloning)

Cloning was conducted using Mighty Cloning Reagent Set (Manufactured by Takara Bio Inc.) in accordance with the attached instruction. As the cloning vector, the attached BAP-treated pUC118 was used.

### (CAPS Analysis)

CAPS (Cleaved Amplified Polymorphic Sequence) analysis was conducted by amplifying PDS3, which was the target sequence, with Prime STAR MAX. In PDS3, a sequence to be cleaved with restriction enzyme BlnI is located immediately before PAM sequence. Hence, when mutation is introduced into the recognition sequence of the restriction enzyme, cleavage of PDS3 by BlnI cannot be conducted. For this reason, it is possible to detect mutants from whether the restriction enzyme is cleaved or not.

### (Sequence Analysis)

DNA sequence reaction was conducted using Big Dye Terminator v3.1 Cycle Sequencing Kit (manufactured by Applied Biosystems) in accordance with the attached instruction. Thereafter, ethanol precipitation was conducted, and after dissolution with formamide, sequencing was conducted in accordance with the instruction of ABI 3130 sequencer. The analysis was conducted using Finch TV.

### (Example 1) Switch on GFP Vector

The present inventors made the concept of a method for expressing an artificial restriction enzyme or the like (genome editing system) in a plant cell and a DNA construct used for the method as shown in Figs. 2 and 3, in order to transiently express an exogenous gene such as an artificial restriction enzyme gene, that induces mutation without incorporating the exogenous gene into the plant genome in preparing a genome-edited plant.

Then, it order to verify that this DNA construct can cleave out cyclic DNA before incorporation into the plant genome, and transiently express the gene of the cyclic DNA, the present inventors first designed the Switch on GFP vector shown in Fig. 4.

A major characteristic of the Switch on GFP vector is that gfp to be used as a reporter gene is incorporated while being divided. That is, the agrobacterium method using this vector introduces T-DNA between LB and RB in the vector into plant and transiently expresses intcre in T-DNA. Subsequently, cleavage occurs due to recombination between loxPs by the enzyme to generate circularization, and as a result, the divided gfp gene is reconstructed, and GFP encoded by the gene is expressed. Hence, it is possible to verify the generation and stability of cyclic DNA by detecting the expression of this GFP. Note that "intcre" and "intron cre" mean Cre recombinase gene in which intron is inserted in the first half of the coding portion.

Then, the Switch on GFP vector thus designed was prepared as follows. First, T-DNA region having MCS (multiple cloning site), RB, OD, and LB×2 was synthesized and cloned into pUC57. Thereafter, nptIII and Ri-Ori of pRI201 vector was amplified with PCR using primers (1) and (2) described below, and the amplified vector was inserted into the EcoRV site.

Lox intron GFP in which thale cress-derived KOR1 intron containing 34 bp loxP sequence was incorporated inside the coding region of gfp was synthesized. The position to incorporate KOR1 intron was determined with consensus region (a g// gtaa gt----t gcaa g// g) of plant intron taken into consideration. Two types of thale cress-derived KOR1 intron containing 34 bp loxP sequence were used as described below.

lox-intron (KOR1) 01 (SEQ ID NO: 1)

GTAAGTCTTCTTTTCCTTTACTCTTTACAGAAATGGTAATCTCAGATATAGTAAat

aacttcgtataatgtatgctatacgaagttatTGGATAAGATCCAAAAATGACACT

TTTAACCAAGATTGTACGAAGATCTTTTTAAACTCCATTTTTTATTTTGACATCTA

AATTGGATTTAACTCGGCCTTGCTGTATTTTGGCAG

lox-intron (KOR1) 02 (SEQ ID NO: 2)

GTAAGTCTTCTTTTCCTTTACTCTTTACAGAAATGGTAATCTCAGATATAGTAATG

GATAAGATCCAAAAATGACACTTTTAACCAAataacttcgtataatgtatgctata

cgaagttatGATTGTACGAAGATCTTTTTAAACTCCATTTTTTATTTTGACATCTA

AATTGGATTTAACTCGGCCTTGCTGTATTTTGGCAG

In the above two types of sequences, capital letters indicate the sequence (171 bp) of thale cress-derived KOR1 intron and lower-case letters indicate loxP sequence (34 bp) .

Introduction of Switch on vector backbone into each restriction enzyme site in MCS shown in Fig. 4 was conducted by cleavage using restriction enzymes and in fusion reaction of PCR amplification fragment, which is summarized as follows.

### lox intron gfp

lox intron gfp was introduced while being divided into two restriction enzyme sites. The portion from 35S promoter to the end of intron was amplified with PCR using primers (3) and (4) described below and was introduced into SmaI site. In addition, the portion from the beginning of intron to HST (heat shock protein terminator) was amplified with PCR using primers (7) and (8) described below and was introduced into Kpn site.

### intron Cre

intron cre having gene-synthesized 35S promoter and HST was amplified using primers (5) and (6) described below and was introduced together with the upstream portion of lox intron gfp into SmaI site.

In addition, in the case where genes described below were inserted into the Switch on GFP vector, the insertion was conducted as follows.

### a-ipt, 35S-ipt

a-ipt having a promoter derived from agrobacterium pO22 line, which was provided by Dr Hiroetsu Wabiko, Akita Prefectural University was amplified using primers (13) and (14) described below and was introduced into EcoRI site. In addition, 35S-ipt having 35S promoter was introduced into the same site using primers (9) and (10) described below.

### iaaH, iaaM

iaaH having 35S promoter and HSP was amplified using primers (15) and (16) described below when introduced with a-ipt, and was amplified using primers (11) and (12) described below when introduced with 35S-ipt, and in each case was introduced into EcoRI site. iaaM having the same promoter and terminator was amplified using primers (17) and (18) described below and was introduced into HindIII site.

### SgRNAs of Cas9 and PDS3 (Single-guide RNAs)

SgRNAs of Cas9 having parsley-derived PCubi ubiquitin promoter and soybean-derived Pea3A terminator and PDS3 having a thale cress-derived AtU-6 promoter, provided by Dr. TOKI Seiichi, the National Institute of Agrobiological Sciences (currently: the National Agriculture and Food Research Organization), were amplified using primers (19) and (20) described below and introduced into NotI site.

Note that all the genes thus inserted were optimized to the frequency of codon usage in tobacco when synthesized. The sequences of the above-described primers are shown below, and positional relations of these sequences are shown in Fig. 6.

### Insertion of Ri-Ori nptIII into EcoRV site

Primer (1) Ri-NPT-F 5' AGCTCGGCACAAGATGATCCTACAAGGTAG 3' (SEQ ID NO: 3, Tm: 59.2°C).
Primer (2) Ri-NPT-R 5' AAAGAGCGTTTAGATGTTGCCATGTTTTAC 3' (SEQ ID NO: 4, Tm: 53.7°C).

### Insertion of lox intron gfp into SmaI site

Primer (3) lox-GUS-F 5' GGATCCGAATTCCCCGTCCCCAGATTAGCC 3' (SEQ ID NO: 5, Tm: 63.3°C).
Primer (4) Out-SmaI(GF-I)-R 5' ACTTCCTGCAGGCCCCTGCCAAAATACAGC 3' (SEQ ID NO: 6, Tm: 61.9°C).

### Insertion of Intron cre into SmaI site

Primer (11) lox-iaaH-F 5' GTCCCCAGATTAGCCTTTTCA 3' (SEQ ID NO: 7, Tm: 48.6°C).
Primer (6)Out-SmaI(HSP-T)-R 5' ACTTCCTGCAGGCTTATCTTTAATCAT 3' (SEQ ID NO: 8, Tm: 56.5°C).

### Insertion of lox intron gfp into KpnI site

Primer (7) Out-KpnI(I-P)-F(end) 5' GATAGTTTAAACTGGGTAAGTCTTCTTTTC 3' (SEQ ID NO: 9, Tm: 52.4°C).
Primer (8)Out-KpnI(HSP-T)-R 5' AAGCTTGCGGCCGCGCTTATCTTTAATCAT 3' (SEQ ID NO: 10, Tm: 57.8°C).

### Insertion of a-Ipt and iaaH into EcoRI site

Primer (9) lox-ipt-F 5' GCTTGGATCCGAATTGTCCCCAGATTAGCC 3' (SEQ ID NO: 11, Tm: 60.6°C).
Primer (10) lox-ipt-R 5' GGCTAATCTGGGGACCTTATCTTTAATCAT 3' (SEQ ID NO: 12, Tm: 55.1°C).
Primer (11) lox-iaaH-F 5' GTCCCCAGATTAGCCTTTTCA 3' (SEQ ID NO: 7, Tm: 48.6°C).
Primer (12) lox-iaaH-R 5' TGGGGACGGGGAATTCTTATCTTTAATCAT 3' (SEQ ID NO: 13, Tm: 55.0°C).

### Insertion of 35S-ipt and iaaH into EcoRI site

Primer (13) lox-ipt-F 5' GCTTGGATCCGAATTGTCCCCAGATTAGCC 3' (SEQ ID NO: 11, Tm: 60.6°C).
Primer (14) lox-SmaI(HSP-T)-R 5' CTTATCTTTAATCATATTCCA 3' (SEQ ID NO: 14, Tm: 38.8°C).
Primer (15) lox-SmaI(HSP-T/ipt)-F 5' ATGATTAAAGATAAGCAACGAAGGTAATGG 3' (SEQ ID NO: 15, Tm: 52.4°C).
Primer (16) lox-EcoRI(a-ipt-T)-R 5' TTATCCTGCAGGCCCACGAATATCCGTGAC 3' (SEQ ID NO: 16, Tm: 60.6°C).

### Insertion of iaaM into HindIII site

Primer (17) Out-HindIII(35S-P)-F 5' GCGCGGCCGCAAGCTGTCCCCAGATTAGCC 3' (SEQ ID NO: 17, Tm: 67.4°C).
Primer (18) lox-HindIII(HSP-T)-R 5' ATTCGGATCCAAGCTCTTATCTTTAATCAT 3' (SEQ ID NO: 18, Tm: 52.4°C).

### Insertion of Cas9 and PDS3 into NotI site of SgRNA

Primer (19) lox-NotI(cas9-P)-F 5' GATGGTACCGCGGCCCTAGCAACGATTGTA 3' (SEQ ID NO: 19, Tm: 61.9°C).
Primer (20) lox-NotI(SgRNA-T)-R 5' GGACAGCTTGCGGCCCAACTTTGTACAAGA 3' (SEQ ID NO: 20, Tm: 60.6°C).

### (Example 2) Verification on Activation of Switch on GFP Vector by Transient Assay

In verifying whether cyclic DNA was generated by the agrobacterium method using the Switch on GFP vector, first, it was checked whether splicing in gfp gene having intron in which loxP was incorporated occurred with no problems in plant cells and GFP was expressed. Specifically, agroinfiltration into Nicotiana benthamiana was conducted using two types of lox intron GFP vectors different in position of loxP sequence shown in Fig. 7 and a GFP vector as control against these.

As a result, GFP fluorescence was observed as shown in Fig. 8 and Fig. 9, and it was verified that splicing of inserted intron occurred with no problems and lox intron GFP gene was activated.

Next, in order to verify whether cyclic DNA was generated by the agrobacterium method using Switch on GFP vector, agroinfiltration into Nicotiana benthamiana was conducted again using two types of Switch on GFP vectors as shown in Fig. 10 and a control GFP vector and observed GFP fluorescence.

As a result, GFP fluorescence was detected as shown in Figs. 11 and 12, and it was verified that cyclic DNA was generated by the agrobacterium method using Switch on GFP vector. However, the fluorescence intensity in the case of introducing Switch on GFP vector was approximately one-tenth of that of control.

Next, agroinfiltration was conducted using Switch on GFP vector in which ipt and iaaH were incorporated as shown in Fig. 13, and GFP fluorescence was observed. As a result, it was revealed that the fluorescence intensity of Switch on GFP vector was improved to 45% of that of control by inserting ipt and iaaH, as shown in Figs. 14 and 15.

Moreover, agroinfiltration was conducted using the Switch on GFP vector, and conducted observation of GFP fluorescence and detection of cyclic DNA with genomic PCR on day 7 and day 35 after the infection. As a result, GFP fluorescence was observed even on day 35 after the infection as shown in Fig. 16, and it was also revealed that cyclic DNA was maintained from the result of genomic PCR as shown in Fig. 17.

The results as described above confirmed that the agroinfiltration using the Switch on GFP vector caused the cleavage of cyclic DNA. Moreover, it was revealed that the cyclic DNA thus cleaved was maintained even on day 35. In addition, it was also revealed that inserting ipt and iaaH into the Switch on GFP vector improved the expression of GFP. Although the reason why the expression is improved is not unclear, the present inventors surmises as follows. That is, it is considered that since the terminator (Ter) and the promoter (35SP) are adjacent to each other, poor expression of GFP occurred; however, the insertion of ipt and iaaH resolved the state to improve the expression of GFP. In addition, there is a possibility that the expression of the inserted ipt gene improved the expression of GFP through the effect of hormones with accelerated biosynthesis.

### (Example 3) Genome Editing Using Switch on GFP Vector

It was verified whether the target gene can be modified (genome editing) without being incorporated into a plant genome as in the above-described concept by using the Switch on GFP vector.

At this time, PDS (phytoene desaturase) gene was selected as the target for genome editing. In addition, as the target sequence in genome editing, the third exon portion (PDS3) of this gene was selected (see Fig. 18).

Then, in attempting the genome editing of this gene, sequence analysis was first conducted in order to determine the PDS3 gene sequence of SR1 tobacco (N. tabacum). Specifically, PDS3 was amplified using the primer sets (1) and (2) shown in Fig. 18. Thereafter, the amplified PDS3 was blunt-end cloned into pUC118, and colony PCR was conducted. As a result, one or two bands were obtained as shown in Fig. 19.

Next, plasmids were extracted from each four colonies from which one or two bands were obtained, and sequence analysis was conducted. As a result, two sequences having different homologies were obtained. This is surmised to be because the genome of SR1 is an amphidiploid (2n = 24) and has N. sylvestris (2n = 12: SS) and N. tometosiformis (2n = 12: TT). Actually, as a result of sequencing one band, the sequence thus obtained had a high homology with that of N. sylvestris. In addition, as a result of sequencing two bands, the sequence had a high homology with that of N. tometosiformis.

The result as described above suggested that when cloning into pUC118 and analyzing the PDS3 gene with colony PCR, in the case where one band was detected, the band was derived from the genome of N. sylvestris, while in the case where two bands were detected, the bands were derived from the genome of N. tometosiformis.

Next, mutagenesis using the Switch on GFP vector was attempted. Note that for mutagenesis, CRISPR/Cas9 system was introduced into the Switch on GFP vector. That is, DNA encoding Cas9 and SgRNA for PDS3 was incorporated into the vector. Moreover, in order to enhance the callus formation ability in hormone-free medium, iaaM was also incorporated to construct Switch on GFP vector for mutagenesis shown in Fig. 20. Then, infection experiment into tobacco (SR1) using the immersion method was conducted to attempt mutagenesis with Cas9.

As a result, as shown in Fig. 21, calli were derived from leaf pieces of SR1 into which the Switch on GFP vector had been introduced. In addition, 2 calli were sampled from each of two experimental plots at random on day 39 and day 45 after the infection, and PDS3 was amplified with PCR using the primers (1) and (2) shown in Fig. 18. Subsequently, CAPS analysis was conducted on the amplified fragments to detect mutagenesis. As a result, as shown in Fig. 22, it was confirmed that mutation had been introduced into the target sequence PDS3 in one callus in each experimental plot.

Moreover, fragments amplified using the primers (1) and (2) shown in Fig. 18 were blunt-end cloned into pUC118 using Mighty Cloning Reagent Set. For colonies obtained by the cloning, the insertion of amplified fragments was confirmed by blue-white screening and colony PCR using the primers (3) and (4) shown in Fig. 18. Then, it was confirmed that mutation had been introduced into the target sequence by subjecting the reaction liquid of the colony PCR to BlnI treatment (see Figs. 23 and 24). In addition, sequence analysis conducted on clone with uncleavage revealed substitution or short deletion of DNA sequence as shown in Table 4 described below.

**[Table 4]**

| | Sequence | SEQ NO: | Mutation | Clone count |
|---|---|---|---|---|
| PDS3 (WT/Target Sequence) | GAGGCAAGAGATGTCCTAGGTGG | 21 | - | - |
| PDS3 (Day 39) | GAGGCAAGAG-------AGGTGG | 22 | 7 nucleotide deletions | 1 |
| | GAGGCAAGAaAgGTggaAGGTGa | 23 | 6 nucleotide substitutions | 1 |
| | GAGGCAAGAGATGTCCTggaTGa | 24 | 4 nucleotide substitutions | 2 |
| PDS3 (Day 45) | GAGGCAAGAGATG---------- | 25 | 10 nucleotide deletions | 4 |
| | GAGGCAAGAGATGTCC----Ttt | 26 | 4 nucleotide deletions, 2 nucleotide substitutions | 2 |

Note that in Sequence in Table 4, "-" indicates a site where the base was deleted from the wild type (WT), and base written in lower-case indicates a site where the base was substituted from the wild type (WT).

As described above, the results of the CAPS analysis and the sequence analysis revealed that the Switch on GFP vector allows the genome editing system to express in plant cells to introduce mutation into the target gene.

Note that the sequences of the primers (1) to (4) shown in Fig. 18 and the primer used in the sequence analysis on the uncleaved clone are as follows.

### Blunt-end cloning of PDS3

Primer (1) NtPDS(1866-1892SG)F 5' ACATATAGGGGTTGCACTCTTCTAAGG 3' (SEQ ID NO: 27, Tm: 54.4°C).
Primer (2) NtPDS(2993-3017c)R 5' CCCAGTCTCATACCAATCTCCATCA 3' (SEQ ID NO: 28, Tm: 53.9°C).

### Colony PCR of PDS3

Primer (3) PDS3-F2(Affrc) 5' GTATTGTCATCAACTGGTTGATTATCTGAGTACC 3' (SEQ ID NO: 29, Tm: 57.0°C).
Primer (4) PDS3-R2(Affrc) 5' CTTTCCATATCTTTTACCTAATATGCTG 3' (SEQ ID NO: 30, Tm: 50.3°C).

### Sequence analysis of PDS3

PUC M4 5' GTTTTCCCAGTCACGAC 3' (SEQ ID NO: 31, Tm: 43.2°C).
PUC RV 5' CAGGAAACAGCTATGAC 3' (SEQ ID NO: 32, Tm: 40.8°C).

### (Example 4) Preparation of Genome-edited Individuals by Introduction of Switch on GFP Vector

Infection experiment to SR1 tobacco was conducted by the agrobacterium method through immersion using the Switch on GFP vector to select recombinant individuals having the cyclic DNA. In addition, in order to improve the selection effect, plant hormone genes to be incorporated into T-DNA and culture conditions were considered, aiming at acceleration of the cell proliferation at the infection.

Specifically, as shown in Fig. 25, mutated Switch on GFP vectors having 3 types of Cas9 in which combinations of plant hormone genes were changed were prepared and used. Note that auxin biosynthesis in hormone-free medium was accelerated by incorporating auxin precursor biosynthesis gene iaaM into vectors having iaaH. In addition, conditions that accelerate auxin biosynthesis were also studied using the MS medium to which NAM (naphthaleneacetamide), which is an analog of auxin precursor, was added without incorporation of iaaM. More specifically, tobacco leaf pieces into which construct A shown in Fig. 25 was introduced were cultured in the MS medium, tobacco leaf pieces into which construct B was introduced were cultured in the MS medium or MS NAM medium, and tobacco leaf pieces into which construct C was introduced were cultured in the MS medium or MS NAM medium.

As a result of conducting infection experiment on 20 leaf pieces in each of the above-described five culture conditions, calli of 18 leaf pieces were obtained (6 leaf pieces in the MS medium of construct A, 2 leaf pieces in the MS medium of construct B, 5 leaf pieces in the MS NAM medium of construct B, and 5 leaf pieces in the MS medium of construct C). Note that no difference in callus formation ability due to the combinations of plant hormone genes in vectors or the presence or absence of NAM in the MS medium was observed.

In addition, as shown in Fig. 26, GFP fluorescence was observed in 5 leaf pieces (2 leaf pieces in the MS medium of construct A, 2 leaf pieces in the MS NAM medium of construct B, and 1 leaf piece in the MS medium of construct C) among 18 leaf pieces in which calli were formed.

Moreover, sites where callus formation had occurred in these leaf pieces were collectively sampled, and genomes were extracted and subjected to PCR analysis using the primer sets shown in Fig. 27. As a result, as shown in Fig. 28, gfp (cyclic DNA) and Cas9 were detected. Furthermore, as in the result shown in Example 3, mutagenesis was also detected by CAPS analysis.

Note that the sequences of the primers (1) to (4) shown in Fig. 28 are as follows.

### Detection of gfp (cyclic DNA)

Primer (1) pRI-GFP F 5' CACTGTTGATACATATGGTGAGCAAGGGCG 3' (SEQ ID NO: 33, Tm: 59.2°C).
Primer (2) pRI-GFP R 5' CTTCATCTTCATAAGTTACTTGTACAGCTC 3' (SEQ ID NO: 34, Tm: 53.7°C).

### Detection of Cas9

Primer (3) FFCas9-113F 5' CCGATAGGCACTCTATCAAG 3' (SEQ ID NO: 35, Tm: 48.0°C).
Primer (4) FFCas9-640R 5' CAGAGAGGATAGCCTTAGCA 3' (SEQ ID NO: 36, Tm: 48.0°C).

### (Example 5) Preparation 2 of Genome-edited Individual by Introduction of Switch on GFP Vector

Like Example 4, infection was made using mutated Switch on GFP vector having a construct shown in B of Fig. 25. As a result of conducting infection experiment on 16 leaf pieces for each, 29 calli were obtained in MS medium. In addition, GFP fluorescence was detected in 12 calli among the 29 calli, and the calli in which GFP fluorescence was detected were selected. Moreover, mutagenesis was detected by CAPS analysis in 3 calli among the 12 calli selected by GFP fluorescence. Mutated calli that were considered to have undergone no random insertion into the genome were obtained.

### (Comparative Example 1) Preparation of Genome-edited Individual by Introduction of Switch on GFP Vector (with no Cre Cassette)

Infection was made by the same method as in Example 4, using mutated Switch on GFP vector that had the construct shown in B of Fig. 25 but did not contain cassette (intcre) for Cre recombinase expression on the outside thereof. As a result of conducting infection experiment on 20 leaf pieces for each, 40 calli were obtained in MS medium. However, no callus having GFP fluorescence was obtained among the 40 calli. Moreover, no callus in which mutagenesis was detected by CAPS analysis was obtained among the 40 calli.

From the results shown in Examples and Comparative Examples as described above, in the present invention, DNA cleaved by Cre recombinase circularizes to enable GFP fluorescence to express. Then, it was found that selection of calli using the fluorescence as an indicator is effective in obtaining mutated calli.

### [Industrial Applicability]

As described so far, the present invention makes it possible to modify the sequence of a target gene without exogenous genes encoding genome editing system and the like being incorporated into the genome in a plant cell, and is thus advantageous in terms of safety. In addition, since the present invention allows a genome editing system to stably express for a certain period of time, it is possible to conduct genome editing with high efficiency. The present invention is therefore useful in breeding of agricultural crops, production of useful materials, cultivar improvement, analysis of functions of target genes, and the like. [Sequence Listing] IBPF19-532WO-J(seq)fin.txt

## Claims

1. A DNA construct for expressing a genome editing system in a plant cell, comprising:
a cleaving target region flanked by a first recombinase recognition site located on a 5' side and a second recombinase recognition site located on a 3' side, wherein
the first recombinase recognition site and the second recombinase recognition site are in the same direction,
a gene encoding a site-specific recombinase that specifically recognizes the first and second recombinase recognition sites is arranged outside of at least one side of the cleaving target region,
the cleaving target region contains: a gene encoding the genome editing system; and a marker gene divided into a 5' region and a 3' region,
the 3' region of the marker gene is adjacent to a 3' terminal of the first recombinase recognition site, the 5' region of the marker gene is adjacent to a 5' terminal of the second recombinase recognition site, and the gene encoding the genome editing system is arranged between the 3' region and the 5' region of the marker gene, and
when the DNA construct is introduced into a plant cell, the site-specific recombinase expresses to cleave out and circularize the cleaving target region, causing the 5' region and the 3' region to bind via the recombinase recognition site, which allows the marker gene to express.

2. The DNA construct according to claim 1, further comprising:
a right boundary sequence (RB) and a left boundary sequence (LB) that are derived from an agrobacterium T-DNA sequence, wherein
the cleaving target region and the gene encoding the site-specific recombinase are arranged in a transfer region flanked by the RB and the LB.

3. The DNA construct according to claim 1 or 2, wherein
the marker gene is a fluorescent protein gene, a luminescent enzyme gene, a chromogenic enzyme gene, or a drug resistance gene.

4. The DNA construct according to any one of claims 1 to 3, wherein
the marker gene is divided into the 5' region and the 3' region in intron.

5. The DNA construct according to any one of claims 1 to 4, further comprising:
an exogenous gene between the 3' region and the 5' region of the marker gene in the cleaving target region.

6. The DNA construct according to claim 5, wherein
the exogenous gene is a phytohormone biosynthetic gene.

7. A method for producing a genome-edited plant cell, the method comprising the steps of:
(1) introducing the DNA construct according to any one of claims 1 to 6 into plant cells; and
(2) selecting a cell in which the DNA construct has been introduced and circularization of the cleaving target region has occurred from cells obtained in the step (1) using expression of the marker gene as an indicator.

8. A method for producing a genome-edited plant, the method comprising the steps of:
(1) introducing the DNA construct according to claim 6 into plant cells;
(2) selecting a cell in which the DNA construct has been introduced and circularization of the cleaving target region has occurred from cells obtained in the step (1) using expression of the marker gene as an indicator; and
(3) culturing the cell obtained in the step (2) to generate plant tissues, and selecting a plant tissue in which morphological change has occurred, by using the morphological change in the plant tissues attributable to expression of the phytohormone biosynthetic gene on the DNA construct as an indicator.
